# EUROPEAN PATENT APPLICATION

(11) **EP 4 538 709 A1**
(43) Date of publication of application: **16.04.2025**
(21) Application number: 23870620.4
(22) Date of filing: 22.09.2023
(51) Int. Cl.: G01N 33/68, G01N 33/74, G01N 33/53, C12N 5/20, C07K 16/00

(54) **MARKER FOR DIAGNOSING MULTIPLE SCLEROSIS AND USE THEREOF**

(30) Priority: 30.09.2022 CN 202211211353
(71) Applicant: Xiamen Innobiomax Biotechnology Co., Ltd., Xiamen, Fujian 361000 (CN); Xiamen Innodx Biotech Co., Ltd, Xiamen, Fujian 361022 (CN); Beijing Tiantan Hospital Capital Medical University, Beijing 100070 (CN)
(72) Inventor: WANG, Haihong, Xiamen, Fujian 361022 (CN); ZHANG, Guojun, Beijing 100050 (CN); TONG, Xunzhang, Xiamen, Fujian 361022 (CN); KE, Qishen, Xiamen, Fujian 361022 (CN); ZOU, Mengwen, Xiamen, Fujian 361022 (CN); SHI, Yijun, Beijing 100050 (CN); JIANG, Wencan, Beijing 100050 (CN); LI, Xiaotong, Beijing 100050 (CN); DING, Yaowei, Beijing 100050 (CN); XU, Ting, Beijing 100050 (CN); LI, Xiangyun, Xiamen, Fujian 361022 (CN); XIONG, Junhui, Xiamen, Fujian 361022 (CN); SUN, Xudong, Xiamen, Fujian 361022 (CN)
(74) Representative: Brann AB
(86) International application number: PCT/CN2023/120646
(87) International publication number: WO 2024/067389

(57) **Abstract**

The present application belongs to the technical field of biomedicine. More specifically, the present application relates to a kit for detecting whether a subject suffers from multiple sclerosis (MS). Furthermore, the present application relates to a kit capable of being used for discriminating MS and neuromyelitis optica spectrum disorders (NMOSD). Further, the present application also relates to a method for screening a reagent or a reagent combination which can be used for detecting whether a subject suffers from MS and can discriminate NMOSD and MS.

## Description

### Technical Field

The present application belongs to the field of biomedical technology. More specifically, the present application relates to a kit for detecting whether a subject suffers from multiple sclerosis (MS), and a kit that can be used to screen for MS and neuromyelitis optica spectrum diseases. (NMOSD) kit. Further, the present application also relates to a method for screening a reagent or reagent combination that can be used to detect whether a subject suffers from MS, and/or that can screen for NMOSD and MS.

### Background Art

Multiple sclerosis (MS) is a chronic autoimmune demyelinating disease with pathological characteristics of discrete and focal myelin loss areas, known as plaques or lesions, in which the plaques can be composed of varying amounts of demyelination, gliosis, inflammation, edema and axonal degeneration. Different theories suggest that environmental factors, autoimmune disorders, and genetic abnormalities are involved in the development of the disorder, but the cause remains unknown.

MS is the second most common central nervous system (CNS) disease among young people after epilepsy, and is one of the most common neurological disorders among young people. According to the latest data from the WHO, there are currently about 2.5 million MS cases worldwide, and the prevalence has increased by 10.4% since 1990, and is still increasing year by year. At present, the sick people are mainly concentrated in North America and Europe, and there is a lack of relevant epidemiological research in Asia. Although there are currently few reported cases, WHO believes that with the application of magnetic resonance imaging (MRI) and the participation of more neurologists, a considerable number of people in Asia will be diagnosed. MS carries a risk of subsequent chronic functional impairment and loss of ability within 10% to 15% of the disease duration. About 10% of patients present with primary progressive MS (PPMS), which is defined as symptoms that gradually worsen throughout the disease process without any clinical relief. After the onset of PPMS, symptoms have been chronically worsening and the prognosis is poor. About 80% to 90% of patients present with relapsing and remitting MS (RRMS), which is defined as a clinical episode (relapse) lasting at least 24 hours followed by partial or complete recovery (remission); wherein 40% of RRMS will develop secondary progressive MS (SPMS), with symptoms worsening and remissions eventually disappearing. MS patients with rapid disease progression can die within a few months, and a few patients have a long remission period and can survive for more than 30 years. Even if the patients survive, more than 60% of patients will eventually lose their ability to move, seriously affecting the patient's quality of life and causing a huge economic burden on society.

Diagnostic criteria for MS have been formulated as early as the 1960s and 1970s, including Schumacher, McAlpine, Rose and other criteria that are mainly based on clinical manifestations. However, they lack imaging and laboratory evidence, making it easy to misdiagnose other diseases as MS. By the 1980s, with the clinical application of imaging diagnostic technology and cerebrospinal fluid immunological detection indicators, such as oligoclonal band (OB), the diagnostic criteria for MS were continuously improved. In 2001, MRI technology was first integrated into the diagnosis of MS, and it was recommended to use "MS", "suspicious MS" and "non-MS" for diagnostic stratification. In 2005, clinical experts launched a revised version based on the 2001 version. The main revisions are: ① emphasis on the importance of multiple evidence of lesion time in diagnosis; ② explanation of the determination of spinal cord lesions and their role in diagnosis; ③ simplification of diagnostic criteria for PPMS. The revised Mcdonald criteria achieve relatively early diagnosis of MS, have higher sensitivity and specificity than those in the past, and can be used in clinical practice and experiments. It is a milestone in the progress of MS clinical diagnosis. However, the revised standards are still relatively complex, and even experienced MS experts are unable to determine whether the diagnosis of some patients meets part of the standards, making it difficult for universal application in clinical practice. In view of the above shortcomings, Polman et al. revised the 2005 version of the standard again in 2010 and launched the 2010 version of the Mcdonald standard. Compared with past standards, its sensitivity and specificity are the same, but the diagnostic process is simplified, the number of MRI examinations required is reduced, and the MRI time and space multiplex criteria have also been modified. The clinical diagnostic criteria are: ① there is evidence of 2 different lesions in the white matter of the central nervous system, with a disease duration of >1 year; ② there is a history of at least > 2 remissions and relapses; ③ there is a chronic and gradually worsening disease duration of at least > 6 months; ④ the age of onset is between 10 and 50 years old; ⑤ the symptoms of neurological lesions cannot be explained by other causes. The modified definition of spatial multiple occurrence requires any one of the following two items: ① there is ≥1 T2 lesion in at least 2 of the 4 typical central CNS demyelination lesion areas (paraventricular, juxtacortex, infratentorial, and spinal cord); ② another clinical attack involving different parts of CNS is imminent. Temporal multiplex requires any one of the following three items: ① asymptomatic gadolinium-enhancing and non-enhancing lesions are present in MRI detection at any time; ② new T2 lesions and/or gadolinium-enhancing lesions are observed in follow-up MRI detection, regardless of the length of time interval from the baseline MRI scan; ③ another clinical attack is imminent. In view of the fact that the above criteria are still complex, the 2017 version of McDoanld is subjected to the revision of criteria again. However, no matter how it is revised, the current diagnosis of MS mainly relies on the understanding of the subject's clinical history, the MRI detection of brain and spinal cord, and the analysis of CSF. MRI is usually used to observe MS lesions in the body, which lacks specificity in the early stage of the disease, requires large-scale equipment and is expensive. CSF immunological examinations such as OB provide evidence of damaged immune or inflammatory responses, but this result is not specific to MS. Others central demyelinating diseases such as neuromyelitis optica or other chronic infections may also occur and cannot be effectively distinguished. Although the McDonald criteria have significantly shortened the diagnosis time, the complexity and cumbersomeness of its diagnostic classification scheme and MRI criteria limit its daily use. Generally speaking, when used in practice, current diagnostic methods are cumbersome and costly, rely on the experience of relevant professionals, and cannot be objectively quantified. At the same time, they cannot conduct large-scale screening, which is not conducive to clinical popularization.

On the other hand, MS and neuromyelitis optica spectrum disorders (NMOSD) have similar clinical manifestations, and the current "China Neuromyelitis Optica Spectrum Disorders Diagnosis and Treatment Guidelines" have collectively referred to neuromyelitis optica (NMO) and NMOSD as NMOSD. In clinical practice, how to effectively distinguish MS and NMOSD is a problem that has always troubled clinicians. NMOSD is a highly relapsed and highly disabling disease, in which more than 90% of patients have a multi-phase disease course that is relatively common in Asia, about 60% of patients relapse within 1 year and 90% of patients relapse within 3 years, and most patients have severe residual diseases such as visual impairment and/or limb dysfunction, dysuria and defecation difficulties, etc. The prognosis of NMOSD is worse than that of MS, in which about half of the patients have severe visual impairment or even blindness in one eye within 5 years, and about 50% of patients with recurrent NMOSD are unable to walk independently 5 years after onset. A special serum biomarker, aquaporin 4 antibody (AQP4-IgG), discovered in 2004, helps distinguish NMOSD from MS. It has since been thought that abnormal immune responses mediated by AQP4-IgG are key factors for the onset of NMOSD, and are important basis for the diagnosis of NMOSD. However, studies have found that nearly 30% of NMOSD patients are still serum AQP4-IgG negative, and most MS patients are also serum AQP4-IgG negative, which leads to problems in clinical diagnosis and identification of NMOSD and MS. In clinical diagnosis, the differential diagnosis of the two diseases is very critical because the corresponding treatment plans are completely different: drugs that are very effective in treating MS (e.g., IFN-β, natalizumab, oral fingolimod, etc.) are not only ineffective in the treatment of NMOSD but also may aggravate the condition of NMOSD. Therefore, trying to distinguish MS and NMOSD is crucial for effective diagnosis and individualized treatment of the diseases.

Therefore, there is a need to develop a new method and kit for detecting MS, as well as a method and kit for distinguishing MS and NMOSD.

### Contents of the Invention

The applicant of the present application unexpectedly discovered during the study of demyelinating diseases that G protein-coupled receptor 37 (GPR37) levels in the samples from patients with demyelinating diseases including multiple sclerosis (MS) were significantly lower than that of the normal human samples. This result suggests that GPR37 plays an important role in the detection of demyelinating diseases including MS. Next, the applicant of the present application evaluated the samples of demyelinating diseases including MS, neuromyelitis optica spectrum disorders (NMOSD), and Guillain-Barré syndrome (GBS), and found that the level of GPR37 in MS patients was significantly lower than that in NMOSD patients, suggesting that the detection of GPR37 could be further used to distinguish MS and NMOSD.

Furthermore, the applicant of the present application found that antibodies with significant distinguishing value between MS patients and normal people all recognize the extracellular segment of GPR37 (1-265aa) or a fragment thereof (101-265aa). Therefore, antibodies or antibody combinations that can specifically bind to GPR37 protein or a fragment thereof (especially a fragment containing the 1-265aa or 101-265aa region) have great application potential in detecting MS or distinguishing MS and NMOSD.

Therefore, in a first aspect, the present application provides a kit for detecting whether a subject suffers from MS and/or differentiating between NMOSD and MS, wherein the kit comprises a reagent or a combination of reagents for determining a level of a biomarker in a biological sample, and the biomarker is GPR37 or a fragment thereof.

In certain embodiments, the level of the biomarker is a protein level or mRNA level of the biomarker.

In certain embodiments, the fragment of GPR37 comprises the amino acid residues at positions 1 to 265 of the amino acid sequence of GPR37.

In certain embodiments, the fragment of GPR37 comprises the amino acid residues at positions 101 to 265 of the amino acid sequence of GPR37.

In certain embodiments, the GPR37 has the amino acid sequence as set forth in SEQ ID NO: 1.

In certain embodiments, the fragment of GPR37 has the amino acid sequence as set forth in SEQ ID NO: 3 or 21.

In certain embodiments, the biological sample is a blood sample (e.g., whole blood, plasma, or serum), excretion (e.g., urine), secretion (e.g., sweat, tears) or a sample of cerebrospinal fluid or tissue or other human source.

In the existing technology, there are already some reagents and corresponding methods for detecting GPR37 or a fragment thereof (for example, Fitzgerald's antibody, its catalog number is 70R-17581), and those skilled in the art are also capable of preparing new reagents or combinations of reagents for detecting GPR37 or fragments thereof through a variety of technologies (e.g., hybridoma technology, for specific methods, see Kohler et al. Nature, 256:495, 1975; phage antibody library technology, for specific methods, see Clackson et al. Nature352:624-628, 1991). In summary, those skilled in the art have the ability to select appropriate reagents and use corresponding methods to detect GPR37 or fragments thereof. Therefore, in this article, the reagent or combination of reagents used to determine the level of GPR37 or its fragments in biological samples contained in the above-mentioned kit is not limited to the reagents or combinations of reagents specifically used in the present application.

In certain embodiments, in the kit as described above, the level of the biomarker is the protein level of the biomarker.

In certain embodiments, the reagent or combination of reagents is used to determine the level of the biomarker in the biological sample by an immunological assay.

In certain embodiments, the immunological assay is selected from the group consisting of ELISA assay, Western blot, surface plasmon resonance, Elispot assay.

In certain embodiments, the reagent or combination of reagents comprises: a first antibody and/or a second antibody that specifically binds to GPR37 or a fragment thereof.

In certain embodiments, in the kit as described above, the level of the biomarker is the level of an mRNA encoding the biomarker.

In certain embodiments, the reagent or combination of reagents is used to determine the level of the biomarker in the biological sample by quantitative PCR.

In certain embodiments, the reagent or combination of reagents comprises a primer and/or probe capable of quantifying the level of the mRNA encoding GPR37 or a fragment thereof.

In certain embodiments, in the kit as described above, the kit comprises: (i) a first antibody or conjugate thereof, or (ii) a second antibody or conjugate thereof, or (iii) their combination; wherein, the first antibody or the second antibody is an antibody or antigen-binding fragment thereof that specifically binds to GPR37 or a fragment thereof.

In certain embodiments, the kit is used to detect whether a subject has MS.

In certain embodiments, the kit is used to detect whether a subject has NMOSD.

In certain embodiments, the kit is used to discriminate between healthy subjects, subjects suffering from MS, and subjects suffering from NMOSD.

In certain embodiments, the kit comprises: a first antibody, a conjugate of the first antibody, a second antibody, a conjugate of the second antibody, or any combination thereof.

In certain embodiments, the kit comprises: a first antibody and/or a conjugate of the first antibody; and a second antibody and/or a conjugate of the second antibody.

In certain embodiments, the kit is used to discriminate a healthy subject from a subject suffering from a demyelinating disease (e.g., MS, NMOSD). In certain embodiments, the kit is used to discriminate a healthy subject from a subject suffering from MS disease. In certain embodiments, the kit is used to discriminate a healthy subject from a subject suffering from NMOSD disease. In certain embodiments, the kit is used to discriminate a subject suffering from MS disease from a subject suffering from NMOSD disease. In certain embodiments, the kit is used to discriminate between healthy subjects, subjects suffering from MS disease, and subjects suffering from NMOSD disease.

In certain embodiments, the first antibody comprises: the following 3 heavy chain CDRs: a VH CDR1 as set forth in SEQ ID NO: 5, a VH CDR2 as set forth in SEQ ID NO: 6, a VH CDR3 as set forth in SEQ ID NO: 7; and the following 3 light chain CDRs: a VL CDR1 as set forth in SEQ ID NO: 8, a VL CDR2 as set forth in SEQ ID NO: 9, a VL CDR3 as set forth in SEQ ID NO: 10.

In certain embodiments, the first antibody comprises: a VH as set forth in SEQ ID NO: 11; and a VL as set forth in SEQ ID NO: 12.

In certain embodiments, the conjugate of the first antibody comprises the first antibody, and a detectable label linked to the first antibody.

In certain embodiments, the detectable label is selected from the group consisting of enzyme (e.g., horseradish peroxidase, alkaline phosphatase), chemiluminescent reagent (e.g., acridinium ester, luminol and derivative thereof, ruthenium derivative), fluorescent dye (e.g., fluorescein, fluorescent protein), radionuclide or biotin.

In certain embodiments, the detectable label is horseradish peroxidase.

In certain embodiments, the second antibody comprises: the following 3 heavy chain CDRs: a VH CDR1 as set forth in SEQ ID NO: 13, a VH CDR2 as set forth in SEQ ID NO: 14, a VH CDR3 as set forth in SEQ ID NO: 15; and, the following 3 light chain CDRs: a VL CDR1 as set forth in SEQ ID NO: 16, a VL CDR2 as set forth in SEQ ID NO: 17, and a VL CDR3 as set forth in SEQ ID NO: 18.

In certain embodiments, the second antibody comprises: a VH as set forth in SEQ ID NO: 19; and, a VL as set forth in SEQ ID NO: 20.

In certain embodiments, the conjugate of the second antibody comprises the second antibody, and a detectable label linked to the second antibody.

In certain embodiments, the detectable label is selected from the group consisting of enzyme (e.g., horseradish peroxidase, alkaline phosphatase), chemiluminescent reagent (e.g., acridinium ester, luminol and derivative thereof, ruthenium derivative), fluorescent dye (e.g., fluorescein, fluorescent protein), radionuclide or biotin.

In certain embodiments, the detectable label is horseradish peroxidase.

In another aspect, the present application provides a method for detecting whether a subject suffers from MS and/or differentiating between NMOSD and MS, the method comprising: providing a reagent for determining the level of a biomarker in a biological sample, the biomarker is GPR37 or a fragment thereof.

On the other hand, the present application also provides a method for detecting whether a subject suffers from MS and/or differentiating between NMOSD and MS by of using GPR37 or a fragment thereof as a biomarker, the method comprises determining the level of a biomarker in a biological sample, and the biomarker is GPR37 or a fragment thereof.

In certain embodiments, the level of the biomarker in the biological sample obtained from the subject is detected, and the level is compared to the level of the biomarker in a healthy person to determine whether the subject has MS or NMOSD. In certain embodiments, the subject has MS or NMOSD if the level of the biomarker in the subject is lower than the level of the biomarker in the healthy person.

In certain embodiments, the levels of biomarker in biological samples obtained from different subjects are detected, and the levels from different subjects are compared to determine whether the subject has MS or NMOSD. In certain embodiments, the subject suffering from a lower level of the biomarkers has MS or NMOSD.

In another aspect, the present application provides a use of a reagent for determining the level of a biomarker in a biological sample in the manufacture of a kit for detecting whether a subject suffers from MS and/or NMOSD and MS; wherein the biomarker is GPR37 or a fragment thereof.

In certain embodiments, the level of the biomarker is a protein level or mRNA level of the biomarker.

In certain embodiments, the fragment of GPR37 comprises the amino acid residues at positions 1 to 265 of the amino acid sequence of GPR37.

In certain embodiments, the fragment of GPR37 comprises the amino acid residues at positions 101 to 265 of the amino acid sequence of GPR37.

In certain embodiments, the GPR37 has the amino acid sequence as set forth in SEQ ID NO: 1.

In certain embodiments, the fragment of GPR37 has the amino acid sequence as set forth in SEQ ID NO: 3 or 21.

In certain embodiments, the biological sample is a blood sample (e.g., whole blood, plasma, or serum), excretion (e.g., urine), secretion (e.g., sweat, tears) or a sample of cerebrospinal fluid or tissue or other human source.

In certain embodiments, the kit is used to discriminate between subjects suffering from MS and subjects suffering from NMOSD.

In certain embodiments, the level of the biomarker is the protein level of the biomarker.

In certain embodiments, the reagent or combination of reagents is used to determine the level of the biomarker in the biological sample by an immunological assay.

In certain embodiments, the immunological assay is selected from the group consisting of ELISA assay, Western blot, surface plasmon resonance, Elispot assay.

In certain embodiments, the reagent or combination of reagents comprises: a first antibody and/or a second antibody that specifically binds to GPR37 or a fragment thereof.

In certain embodiments, the level of the biomarker is the level of the mRNA encoding the biomarker.

In certain embodiments, the reagent or combination of reagents is used to determine the level of the biomarker in the biological sample by quantitative PCR.

In certain embodiments, the reagent or combination of reagents comprises a primer and/or probe capable of quantifying the level of the mRNA encoding GPR37 or fragments thereof.

In certain embodiments, in use as described above, the kit comprises: (i) a first antibody or conjugate thereof, or (ii) a second antibody or conjugate thereof, or (iii) their combination; wherein, the first antibody or the second antibody is an antibody or an antigen-binding fragment thereof that specifically binds to G protein-coupled receptor 37 (GPR37) or a fragment thereof.

In certain embodiments, the kit is used to discriminate between healthy subjects, subjects suffering from MS, and subjects suffering from NMOSD.

In certain embodiments, the kit is used to discriminate a healthy subject and a subject suffering from a demyelinating disease (e.g., MS, NMOSD). In certain embodiments, the kit is used to discriminate a healthy subject from a subject suffering from MS disease. In certain embodiments, the kit is used to discriminate a healthy subject from a subject suffering from NMOSD disease. In certain embodiments, the kit is used to discriminate a subject suffering from MS disease and a subject suffering from NMOSD disease. In certain embodiments, the kit is used to discriminate between healthy subjects, subjects suffering from MS disease, and subjects suffering from NMOSD disease.

In certain embodiments, the first antibody comprises: the following 3 heavy chain CDRs: a VH CDR1 as set forth in SEQ ID NO: 5, a VH CDR2 as set forth in SEQ ID NO: 6, a VH CDR3 as set forth in SEQ ID NO: 7; and, the following 3 light chain CDRs: a VL CDR1 as set forth in SEQ ID NO: 8, a VL CDR2 as set forth in SEQ ID NO: 9, and a VL CDR3 as set forth in SEQ ID NO: 10.

In certain embodiments, the first antibody comprises: a VH as set forth in SEQ ID NO: 11; and a VL as set forth in SEQ ID NO: 12.

In certain embodiments, the conjugate of the first antibody comprises the first antibody, and a detectable label linked to the first antibody.

In certain embodiments, the detectable label is selected from the group consisting of enzyme (e.g., horseradish peroxidase, alkaline phosphatase), chemiluminescent reagent (e.g., acridinium ester, luminol and derivative thereof, ruthenium derivative), fluorescent dye (e.g., fluorescein, fluorescent protein), radionuclide or biotin.

In certain embodiments, the detectable label is horseradish peroxidase.

In certain embodiments, the second antibody comprises: the following 3 heavy chain CDRs: a VH CDR1 as set forth in SEQ ID NO: 13, a VH CDR2 as set forth in SEQ ID NO: 14, a VH CDR3 as set forth in SEQ ID NO: 15; and, the following 3 light chain CDRs: a VL CDR1 as set forth in SEQ ID NO: 16, a VL CDR2 as set forth in SEQ ID NO: 17, a VL CDR3 as set forth in SEQ ID NO: 18.

In certain embodiments, the first antibody comprises: a VH as set forth in SEQ ID NO: 19; and a VL as set forth in SEQ ID NO: 20.

In certain embodiments, the conjugate of the second antibody comprises the second antibody, and a detectable label linked to the second antibody.

In certain embodiments, the detectable label is selected from the group consisting of enzyme (e.g., horseradish peroxidase, alkaline phosphatase), chemiluminescent reagent (e.g., acridinium ester, luminol and derivative thereof, ruthenium derivative), fluorescent dye (e.g., fluorescein, fluorescent protein), radionuclide or biotin.

In certain embodiments, the detectable label is horseradish peroxidase.

In another aspect, the present application provides a method for screening a reagent or a combination of reagents that can be used to detect whether a subject suffers from MS, and/or can discriminate between NMOSD and MS, the method comprising:
(1) providing a biological sample containing GPR37 or a fragment thereof obtained from a subject, and contacting a reagent or combination of reagents with the biological sample;
(2) detecting whether the reagent or combination of reagents forms a complex with GPR37 or a fragment thereof contained in the biological sample;
(3) determining that if the complex is formed in step (2), the reagent or combination of reagents can be used to detect whether the subject suffers from MS, and/or can discriminate between NMOSD and MS.

In certain embodiments, the fragment of GPR37 comprises the amino acid residues at positions 1 to 265 of the amino acid sequence of GPR37.

In certain embodiments, the fragment of GPR37 comprises the amino acid residues at positions 101 to 265 of the amino acid sequence of GPR37.

In certain embodiments, the GPR37 has the amino acid sequence as set forth in SEQ ID NO: 1.

In certain embodiments, the fragment of GPR37 has the amino acid sequence as set forth in SEQ ID NO: 3 or 21.

In certain embodiments, the biological sample is a blood sample (e.g., whole blood, plasma, or serum), excretion (e.g., urine), secretion (e.g., sweat, tears) or a sample of cerebrospinal fluid or tissue or other human source.

In certain embodiments, the reagent or combination of reagents is used to discriminate between subjects having MS and subjects having NMOSD.

In certain embodiments, the reagent or combination of reagents is an antibody or conjugate thereof capable of specifically binding to GPR37 or a fragment thereof.

In certain embodiments, the conjugate comprises the antibody, and a detectable label linked to the antibody.

In certain embodiments, the detectable label is selected from the group consisting of enzyme (e.g., horseradish peroxidase, alkaline phosphatase), chemiluminescent reagent (e.g., acridinium ester, luminol and derivative thereof, ruthenium derivative), fluorescent dye (e.g., fluorescein, fluorescent protein), radionuclide or biotin.

In certain embodiments, the detectable label is horseradish peroxidase.

### Definition of Terms

In the present invention, unless otherwise stated, scientific and technical terms used herein have the meanings commonly understood by those skilled in the art. Moreover, the procedures used in this article such as molecular genetics, nucleic acid chemistry, chemistry, molecular biology, biochemistry, cell culture, microbiology, cell biology, genomics and recombinant DNA are routine procedures widely used in the corresponding fields. Meanwhile, in order to better understand the present invention, definitions and explanations of relevant terms are provided below.

As used herein, the term "demyelinating disease" refers to a neurological disease in which demyelination of nerves is the main or primary lesion while damage to axons, cell bodies, and glia is relatively minor. Among MS, NMOSD (the current "China Neuromyelitis Optica Spectrum Disorders Diagnosis and Treatment Guidelines" has collectively referred to neuromyelitis optica (NMO) and neuromyelitis optica spectrum disorders (NMOSD) as NMOSD) and GBS and the like, MS and NMOSD have similar clinical manifestations, so that the distinction between MS and NMOSD has always been a difficult problem to solve.

As used herein, the terms "wild" or "natural" are used interchangeably. When these terms are used to describe a nucleic acid molecule, polypeptide or protein, it means that the nucleic acid molecule, polypeptide or protein exists in nature, is found in nature, and has not undergone any artificial modification or processing. As used herein, natural GPR37 protein refers to a naturally occurring, biologically active GPR37 protein. Those skilled in the art can easily obtain the amino acid sequence of GPR37 protein from various public databases (e.g., GenBank database). For example, the amino acid sequence of natural GPR37 protein can be shown as SEQ ID NO: 1.

As used herein, when referring to the amino acid sequence of natural GPR37 protein, it is described using the sequence as set forth in SEQ ID NO: 1. For example, the expression "amino acids at positions 101 to 265 of the natural GPR37 protein" refers to the amino acids at positions 101 to 265 of the protein as set forth in SEQ ID NO: 1. However, those skilled in the art understand that the natural GPR37 protein can have multiple versions that have substantially the same primary structure (i.e., amino acid sequence) and higher-order structure (i.e., spatial structure), as well as substantially the same biological function, but they can still have slight differences in amino acid sequence from each other. Therefore, in the present application, the natural GPR37 protein is not limited to the protein as set forth in SEQ ID NO: 1, but is intended to cover all known natural GPR37 proteins. Therefore, in the present application, the term "natural GPR37 protein" shall include various naturally occurring GPR37 proteins with biological functions, including, for example, the GPR37 protein as set forth in SEQ ID NO: 1 and its naturally occurring variants. Moreover, when describing the amino acid position of GPR37 protein, it comprises not only the specific amino acid position in SEQ ID NO: 1, but also the amino acid position corresponding to the specific amino acid position in its natural variant. For example, the expression "amino acids at positions 101 to 265 of GPR37 protein" comprises the amino acids at positions 101 to 265 of SEQ ID NO: 1, and the corresponding amino acid positions in natural variants thereof. According to the present application, the expression "corresponding amino acid positions" refers to amino acid positions in the compared sequences that are equivalent when the sequences are optimally aligned, that is, when the sequences are aligned to obtain the highest percent identity. In certain embodiments, the GPR37 protein has a sequence as set forth in SEQ ID NO: 1, when the expression "corresponding to amino acids at positions 101 to 265 of GPR37 protein" refers to amino acids at positions in a sequence that are equivalent to the positions 101 to 265 of SEQ ID NO: 1 when the sequence is optimally aligned with SEQ ID NO: 1, that is, when the sequence is aligned with SEQ ID NO: 1 to obtain the highest percent identity.

As used herein, the term "identity" is used to refer to the match of sequences between two polypeptides or between two nucleic acids. When a position in both sequences being compared is occupied by the same base or amino acid monomer subunit (for example, a position in each of two DNA molecules is occupied by adenine, or a position in each of two polypeptides is occupied by lysine), then the molecules are identical at that position. "Percent identity" between two sequences is a function of the number of matching positions common to the two sequences divided by the number of positions compared × 100. For example, if 6 out of 10 positions of two sequences match, then the two sequences have an identity of 60%. For example, the DNA sequences CTGACT and CAGGTT have an identity of 50% (matching at 3 out of 6 total positions). Typically, comparisons are made when two sequences are aligned to yield maximum identity. Such alignment can be accomplished using, for example, the method of Needleman et al.

(1970) J. Mol. Biol. 48:443-453, which can be conveniently performed by a computer program such as the Align program (DNAstar, Inc.). It is also possible to use the algorithm of E. Meyers and W. Miller (Comput. Appl Biosci., 4:11-17 (1988)), which has been integrated into the ALIGN program (version 2.0), to determine the percent identity between two amino acid sequences by the PAM120 weight residue table using a gap length penalty of 12 and a gap penalty of 4. Alternatively, the algorithm of Needleman and Wunsch (J MoI Biol. 48:444-453

(1970)), which has been integrated into the GAP program in the GCG package (available at www.gcg.com), can be used to determine the percent identity between two amino acid sequences using the Blossum 62 matrix or PAM250 matrix and a gap weight of 16, 14, 12, 10, 8, 6, or 4, and a length weight of 1, 2, 3, 4, 5, or 6.

As used herein, the term "antibody" refers to an immunoglobulin molecule typically composed of two pairs of polypeptide chains, each pair having a light chain (LC) and a heavy chain (HC). Antibody light chains can be classified into κ (kappa) and λ (lambda) light chains. Heavy chains can be classified as µ, δ, γ, α, or ε, and define the antibody's isotype as IgM, IgD, IgG, IgA, and IgE, respectively. Within the light and heavy chains, the variable and constant regions are connected by a "J" region of approximately 12 or more amino acids, and the heavy chain also contains a "D" region of approximately 3 or more amino acids. Each heavy chain consists of a heavy chain variable region (VH) and a heavy chain constant region (CH). The heavy chain constant region consists of 3 domains (CH1, CH2 and CH3). Each light chain consists of a light chain variable region (VL) and a light chain constant region (CL). The light chain constant region consists of one domain, CL. The constant domain is not directly involved in the binding of antibody to antigen, but exhibits a variety of effector functions, such as mediating the interaction of immunoglobulin with host tissues or factors, including the binding of various cells of the immune system (e.g., effector cells) to the first component of the classical complement system (C1q). The VH and VL regions can also be subdivided into regions of high variability called complementarity determining regions (CDRs), interspersed with more conservative regions called framework regions (FRs). Each VH and VL consists of 3 CDRs and 4 FRs arranged from the amino terminus to the carboxyl terminus in the following order: FR1, CDR1, FR2, CDR2, FR3, CDR3, FR4. The variable regions (VH and VL) of each heavy chain/light chain pair respectively form the antigen-binding site. The assignment of amino acids to regions or domains can follow the definitions of Kabat, Sequences of Proteins of Immunological Interest (National Institutes of Health, Bethesda, Md. (1987 and 1991)), or Chothia & Lesk (1987) J. Mol. Biol. 196 :901-917; Chothia et al. (1989) Nature 342:878-883.

As used herein, the term "complementarity determining region" or "CDR" refers to the amino acid residues in variable region of an antibody that are responsible for antigen binding. The variable regions of the heavy chain and light chain each contain three CDRs, named CDR1, CDR2 and CDR3. The precise boundaries of these CDRs can be defined according to various numbering systems known in the art, such as the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883), or the IMGT numbering system (Lefranc et al. al., Dev. Comparat. Immunol. 27:55-77, 2003). For a given antibody, one skilled in the art will readily identify the CDRs defined by each numbering system. Moreover, the correspondence between different numbering systems is well known to those skilled in the art (for example, see Lefranc et al., Dev. Comparat. Immunol. 27:55-77, 2003).

As used herein, the terms "monoclonal antibody", "McAb" and "mAb" have the same meaning and are used interchangeably, and refer to an antibody or a fragment of antibody from a group of highly homologous antibody molecules, that is, a group of identical antibody molecules except for natural mutations that may occur spontaneously. Monoclonal antibodies are highly specific for a single epitope on the antigen. Polyclonal antibodies are relative to monoclonal antibodies, which usually contain at least two or more different antibodies, and these different antibodies usually recognize different epitopes on the antigen. Furthermore, the modifier "monoclonal" merely indicates that the antibody is characterized as being obtained from a highly homologous population of antibodies and is not construed as requiring any specific method to prepare the antibody.

Monoclonal antibodies of the present invention can be prepared by a variety of techniques, such as hybridoma technology (see, for example, Kohler et al. Nature, 256:495, 1975), recombinant DNA technology (see, for example, U.S. Patent Application 4,816,567), or phage Antibody library technology (see, for example, Clackson et al. Nature 352: 624-628, 1991, or Marks et al. J. Mol. Biol. 222: 581-597, 1991).

As used herein, the term "subject" refers to a mammal, such as a primate mammal, such as a human. In certain embodiments, the mammal is selected from the group consisting of human, mouse, pig, rabbit, sheep, and monkey.

### Beneficial effects of the present invention

The present application provides a kit for detecting whether a subject suffers from MS and/or differentiating between NMOSD and MS, wherein the kit comprises a reagent or a combination of reagents (e.g., containing antibodies 5A2 and/or 6D2) for determining the level of a biomarker in a biological sample, and the biomarker is GPR37 or a fragment thereof. The antibody or antibody pair has high detection sensitivity and can greatly reduce the amount of sample used. Currently, the diagnosis rate of demyelinating diseases (e.g., MS, NMOSD) in China is low and the number of samples is small. The small number of samples greatly limits the development of clinical research, while high-sensitivity reagents can save the amount of samples, facilitate multiple and repeated verification and other research work, and provide convenience for clinical workers.

Furthermore, the applicant provided the use of the key segments of the above-mentioned biomarker (i.e., amino acids at positions 1-265 and 101-265 of GPR37) in detection, and experimentally confirmed that the antibody or paired antibodies targeting to this key segment (e.g., antibodies 5A2 and/or 6D2) had outstanding detection and discrimination effects (i.e., could effectively detect and discriminate the levels of GPR37 in different subjects); could discriminate MS patients from normal subjects; and could also discriminate between patients with specific demyelinating diseases, for example, NMOSD patients and MS patients).

The embodiments of the present invention will be described in detail below with reference to the accompanying drawings and examples, but those skilled in the art will understand that the following drawings and examples are only used to illustrate the present invention and do not limit the scope of the present invention. Various objects and advantageous aspects of the present invention will become apparent to those skilled in the art from the accompanying drawings and the following detailed description of preferred embodiments.

### Brief Description of the Drawings

Fig. 1 shows the identification results of the prepared monoclonal antibodies running 12% SDS-PAGE gel. Lane 1: Marker, Lane 2: monoclonal antibody 4A1, Lane 3: monoclonal antibody 4C10, Lane 4: monoclonal antibody 4H10- 2, Lane 5: mAb 5A2, Lane 6: mAb 5A3, Lane 7: mAb 5A7, Lane 8: Marker, Lane 9: mAb 5A12, Lane 10: mAb 5B11, Lane 11: mAb 5H11, Lane 12: mAb 6D1, Lane 13: mAb 6D2, Lane 14: mAb 6D3.
Fig. 2 shows the detection results of the antibody pair 5A3-5A2 on MS and normal human (control) serum samples.
Fig. 3 shows the detection results of the antibody pair 5A12-5A2 on MS and normal human (control) serum samples.

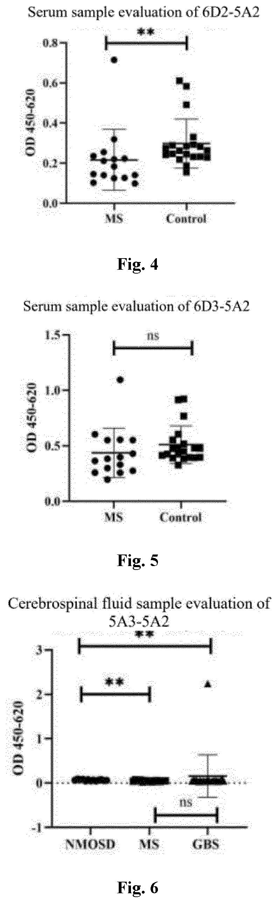

Fig. 4 shows the detection results of the antibody pair 6D2-5A2 on MS and normal human (control) serum samples.
Fig. 5 shows the detection results of the antibody pair 6D3-5A2 on MS and normal human (control) serum samples.
Fig. 6 shows the detection results of antibody pair 5A3-5A2 on cerebrospinal fluid samples from patients with NMOSD, MS and GBS.
Fig. 7 shows the detection results of antibody pair 5A12-5A2 on cerebrospinal fluid samples from patients with NMOSD, MS and GBS.
Fig. 8 shows the detection results of antibody pair 6D2-5A2 on cerebrospinal fluid samples from patients with NMOSD, MS and GBS.
Fig. 9 shows the detection results of antibody pair 6D3-5A2 on cerebrospinal fluid samples from patients with NMOSD, MS and GBS.
Fig. 10 shows the ROC evaluation results of antibody pair 5A12-5A2 on MS and normal humans.
Fig. 11 shows the ROC evaluation results of the antibody pair 6D2-5A2 on MS and normal humans.
Fig. 12 shows the ROC evaluation results of antibody pair 6D2-5A2 on NMOSD and MS patients.
Fig. 13 shows the identification results of protein TF 101-265 running the gel, in which Lane 1: Marker, and Lane 2: TF 101-265aa.
Fig. 14 shows the detection linearity results of the antibody pair 6D2-5A2.

### Sequence Information

Information on partial sequences involved in the present invention is provided in Table 1 below.

**Table 1: Description of sequences**

| SEQ ID NO: | Description | Sequence |
|---|---|---|
| 1 | GPR37 full-length amino acid sequence | MRAPGALLARMSRLLLLLLLKVSASSALGVAPASRNETCLGESC APTVIQRRGRDAWGPGNSARDVLRARAPREEQGAAFLAGPSW DLPAAPGRDPAAGRGAEASAAGPPGPPTRPPGPWRWKGARGQE PSETLGRGNPTALQLFLQISEEEEKGPRGAGISGRSQEQSVKTVP GASDLFYWPRRAGKLQGSHHKPLSKTANGLAGHEGWTIALPGR ALAQNGSLGEGIHEPGGPRRGNSTNRRVRLKNPFYPLTQESYGA YAVMCLSVVIFGTGIIGNLAVMCIVCHNYYMRSISNSLLANLAF WDFLIIFFCLPLVIFHELTKKWLLEDFSCKIVPYIEVASLGVTTFT LCALCIDRFRAATNVQMYYEMIENCSSTTAKLAVIWVGALLLA LPEVVLRQLSKEDLGFSGRAPAERCIIKISPDLPDTIYVLALTYDS ARLWWYFGCYFCLPTLFTITCSLVTARKIRKAEKACTRGNKRQI QLESQMNCTVVALTILYGFCIIPENICNIVTAYMATGVSQQTMD LLNIISQFLLFFKSCVTPVLLFCLCKPFSRAFMECCCCCCEECIQK SSTVTSDDNDNEYTTELELSPFSTIRREMSTFASVGTHC |
| 2 | GPR37 full-length nucleotide sequence | ATGCGCGCTCCGGGTGCTCTGCTGGCCCGTATGAGCCGCCTG CTGCTGCTGTTACTGCTGAAAGTGAGTGCCAGCAGTGCCCTG GGTGTGGCACCGGCTAGCCGTAATGAAACCTGCCTGGGTGAA AGTTGTGCCCCGACCGTTATTCAGCGCCGCGGCCGTGATGCA TGGGGCCCTGGTAATAGCGCCCGCGATGTTCTGCGCGCACGT GCCCCTCGTGAAGAACAGGGTGCCGCATTTCTGGCAGGCCCG AGCTGGGATCTGCCGGCAGCACCTGGTCGCGATCCGGCTGCA GGTCGTGGCGCAGAAGCAAGTGCCGCAGGCCCGCCTGGCCCT CCTACAAGACCGCCTGGCCCGTGGCGTTGGAAAGGCGCACGT GGCCAGGAACCGAGCGAAACCCTGGGTCGTGGTAATCCGAC CGCACTGCAGCTGTTTCTGCAGATTAGTGAAGAAGAAGAAAA AGGTCCGCGCGGTGCCGGTATTAGTGGTCGCAGTCAGGAACA GAGCGTGAAAACCGTTCCGGGCGCCAGTGATCTGTTTTATTG GCCGCGCCGTGCAGGTAAACTGCAGGGCAGTCATCATAAACC GCTGAGCAAAACCGCAAATGGTCTGGCAGGTCATGAAGGCT GGACCATTGCCCTGCCGGGTCGCGCACTGGCACAGAATGGTA GCCTGGGTGAAGGTATTCATGAACCGGGTGGTCCGCGCCGTG GCAATAGCACCAATCGCCGCGTGCGCCTGAAAAATCCGTTTT ATCCGCTGACCCAGGAAAGCTATGGCGCATACGCTGTTATGT GCCTGAGTGTTGTTATTTTTGGTACAGGCATTATTGGTAATCT GGCAGTTATGTGCATTGTTTGCCATAATTATTACATGCGCAGT |
| | | ATTAGCAATAGTCTGCTGGCCAATCTGGCATTTTGGGATTTTC TGATTATTTTCTTTTGCCTGCCGCTGGTTATTTTTCATGAACTG ACCAAAAAATGGCTGCTGGAAGATTTTAGCTGTAAAATTGTG CCGTATATTGAAGTGGCAAGCCTGGGCGTGACCACCTTTACC CTGTGTGCCCTGTGTATTGATCGTTTTCGTGCAGCAACCAATG TTCAGATGTATTATGAAATGATCGAGAATTGCAGTAGTACCA CCGCAAAACTGGCCGTTATTTGGGTTGGCGCCCTGCTGCTGG CACTGCCGGAAGTTGTTCTGCGCCAGCTGAGTAAAGAAGATT TGGGTTTTAGCGGTCGTGCCCCGGCAGAACGTTGCATTATTA AGATTAGTCCGGATCTGCCGGATACCATCTATGTGCTGGCAC TGACCTATGATAGCGCACGCCTGTGGTGGTATTTTGGTTGTTA TTTTTGTCTGCCGACCCTGTTTACCATTACCTGTAGCCTGGTT ACCGCACGTAAAATTCGCAAAGCAGAAAAAGCATGCACCCG CGGCAATAAGCGTCAGATTCAGCTGGAAAGCCAGATGAATT GTACCGTTGTTGCACTGACCATTCTGTATGGTTTTTGTATTAT TCCGGAAAATATCTGTAACATCGTGACCGCCTATATGGCAAC CGGCGTGAGCCAGCAGACCATGGATCTGCTGAATATTATTAG TCAGTTTCTGCTGTTTTTCAAGAGCTGTGTTACCCCGGTGCTG CTGTTTTGCCTGTGTAAACCGTTTAGTCGTGCATTCATGGAAT GTTGCTGTTGTTGTTGTGAAGAATGCATTCAGAAAAGTAGTA CCGTTACCAGCGATGATAATGATAATGAATATACCACCGAAC TGGAACTGAGCCCGTTTAGCACCATTCGTCGCGAAATGAGCA CCTTTGCAAGTGTGGGCACCCATTGT |
| 3 | GPR37 extracellular segment 1-265aa amino acid sequence | MRAPGALLARMSRLLLLLLLKVSASSALGVAPASRNETCLGESC APTVIQRRGRDAWGPGNSARDVLRARAPREEQGAAFLAGPSW DLPAAPGRDPAAGRGAEASAAGPPGPPTRPPGPWRWKGARGQE PSETLGRGNPTALQLFLQISEEEEKGPRGAGISGRSQEQSVKTVP GASDLFYWPRRAGKLQGSHHKPLSKTANGLAGHEGWTIALPGR ALAQNGSLGEGIHEPGGPRRGNSTNRRVRLKNPFYPLTQESYGA YAVM |
| 4 | GPR37 extracellular segment 1-265aa nucleotide sequence | ATGCGCGCTCCGGGTGCTCTGCTGGCCCGTATGAGCCGCCTG CTGCTGCTGTTACTGCTGAAAGTGAGTGCCAGCAGTGCCCTG GGTGTGGCACCGGCTAGCCGTAATGAAACCTGCCTGGGTGAA AGTTGTGCCCCGACCGTTATTCAGCGCCGCGGCCGTGATGCA TGGGGCCCTGGTAATAGCGCCCGCGATGTTCTGCGCGCACGT GCCCCTCGTGAAGAACAGGGTGCCGCATTTCTGGCAGGCCCG AGCTGGGATCTGCCGGCAGCACCTGGTCGCGATCCGGCTGCA GGTCGTGGCGCAGAAGCAAGTGCCGCAGGCCCGCCTGGCCCT CCTACAAGACCGCCTGGCCCGTGGCGTTGGAAAGGCGCACGT GGCCAGGAACCGAGCGAAACCCTGGGTCGTGGTAATCCGAC CGCACTGCAGCTGTTTCTGCAGATTAGTGAAGAAGAAGAAAA AGGTCCGCGCGGTGCCGGTATTAGTGGTCGCAGTCAGGAACA GAGCGTGAAAACCGTTCCGGGCGCCAGTGATCTGTTTTATTG GCCGCGCCGTGCAGGTAAACTGCAGGGCAGTCATCATAAACC GCTGAGCAAAACCGCAAATGGTCTGGCAGGTCATGAAGGCT GGACCATTGCCCTGCCGGGTCGCGCACTGGCACAGAATGGTA GCCTGGGTGAAGGTATTCATGAACCGGGTGGTCCGCGCCGTG GCAATAGCACCAATCGCCGCGTGCGCCTGAAAAATCCGTTTT ATCCGCTGACCCAGGAAAGCTATGGCGCATACGCTGTTATG |
| 5 | 6D2 heavy chain variable region CDR1 | GFIFSSQA |
| 6 | 6D2 heavy chain variable region CDR2 | ISSGGSYT |
| 7 | 6D2 heavy chain variable region CDR3 | ARQRDFGRYYGYDPFFDF |
| 8 | 6D2 light chain variable region CDR1 | SSISSNY |
| 9 | 6D2 light chain variable region CDR2 | RTS |
| 10 | 6D2 light chain variable region CDR3 | QQGSSVPRIT |
| 11 | 6D2 heavy chain variable region | EVMLVESGGGLVKPGGSLKLSCAASGFIFSSQAMSWVRQIPEKR LEWVATISSGGSYTYYPDSVKGRFTISRDNAKNTLYLQMSSLTS EDTAMYYCARQRDFGRYYGYDPFFDFWGQGTTLTF |
| 12 | 6D2 light chain variable region | EIVLTQSPTTMAASPGEKITITCSASSSISSNYLHWYQQKPGFSPK LLIYRTSNLPSGVPARFSGSGSGTSYSLTIGTMEAEDVATYYCQQ GSSVPRITFGAGTKLE |
| 13 | 5A2 heavy chain variable region CDR1 | GFIFSDYY |
| 14 | 5A2 heavy chain variable region CDR2 | IRNKAKRYTT |
| 15 | 5A2 heavy chain variable region CDR3 | ARDTSDYDRFAY |
| 16 | 5A2 light chain variable region CDR1 | QSLVHRNGNTY |
| 17 | 5A2 light chain variable region CDR2 | KVS |
| 18 | 5A2 light chain variable region CDR3 | SQSTLLPYT |
| 19 | 5A2 heavy chain variable region | EVKLVESGGGLVQPGGSLRLSCATSGFIFSDYYMSWVRQPPGK ALEWLGYIRNKAKRYTTEYNASVKGRFTISRDNSQNILYLQMN TLRAEDSATYYCARDTSDYDRFAYWGQGTLVTVSA |
| 20 | 5A2 light chain variable region | DIVMTQTPLSLPVSLGDHASLSCKSSQSLVHRNGNTYLHWFLQK PGQSPKLLIYKVSNRFSGVPDRFSGSGSGTDFTLKISRVEAADLG VYFCSQSTLLPYTFGGGTKLEIK |
| 21 | GPR37 extracellular segment 101-265aa amino acid sequence | GAEASAAGPPGPPTRPPGPWRWKGARGQEPSETLGRGNPTALQ LFLQISEEEEKGPRGAGISGRSQEQSVKTVPGASDLFYWPRRAG KLQGSHHKPLSKTANGLAGHEGWTIALPGRALAQNGSLGEGIH EPGGPRRGNSTNRRVRLKNPFYPLTQESYGAYAVM |

### Specific Models for Carrying Out the Invention

The present invention will now be described with reference to the following examples which are intended to illustrate but not to limit the present invention.

Unless otherwise indicated, the experiments and methods described in the examples were performed essentially according to conventional methods well known in the art and described in various references. For example, conventional techniques such as immunology, biochemistry, chemistry, molecular biology, microbiology, cell biology, genomics and recombinant DNA used in the present invention can be found in Sambrook, Fritsch (Fritsch and Maniatis, MOLECULAR CLONING: A LABORATORY MANUAL, 2nd ed. (1989); CURRENT PROTOCOLS IN MOLECULAR BIOLOGY (edited by F.M. Ausubel et al., (1987)); "METHODS IN ENZYMOLOGY" series (Academic Publishing Company): "PCR 2: A PRACTICAL" APPROACH (edited by M.J. MacPherson, B.D. Hames and G.R. Taylor (1995)), "Antibodies" edited by Harlow and Lane (1988): Laboratory Manual" (ANTIBODIES, A LABORATORY MANUAL), and "ANIMAL CELL CULTURE" (edited by R.I. Freshney (1987)).

In addition, if the specific conditions were not specified in the examples, the conventional conditions or the conditions recommended by the manufacturer shall be followed. If the manufacturer of the reagents or instruments used is not indicated, they are all conventional products that can be purchased commercially. Those skilled in the art will appreciate that the examples describe the present invention by way of example and are not intended to limit the scope of the present invention as claimed. All publications and other references mentioned herein are incorporated by reference in their entirety.

### Example 1. Preparation of recombinant GPR37-related protein

The amino acid sequence of full-length human GPR37 (Gene Bank: NM_005302.5) was set forth in SEQ ID NO: 1, and its full-length nucleotide sequence was set forth in SEQ ID NO: 2. The human GPR37 full-length gene was synthesized by General Biotech Co., and integrated into pET-32a vector, and the PCR primers were completed by General Biotech Co. The pET-32a-GPR37-1-613 full-length antigen (hereinafter referred to as Antigen 1) was expressed and purified using the *Escherichia coli* expression system.

The target fragment plasmid of Antigen 1 was recovered through enzyme digestion, and the target fragment was connected to the pCold-TF expression vector to further express and prepare pCold-TF-GPR37-1-613 full-length antigen (hereinafter referred to as Antigen 2).

Using molecular cloning technology, using the target fragment of Antigen 1 as a template, primers were designed to amplify the extracellular segment protein of GRP37, namely 1-265aa (the sequence of amino acids at positions 1 to 265 of GRP37 as set forth in SEQ ID NO: 3, and its nucleotide sequence was set forth in SEQ ID NO: 4), the target fragment was recovered, and connected to the pCold-TF expression vector to prepare pCold-TF-GPR37-1-265 extracellular segment protein (hereinafter referred to as Antigen 3).

The purified Antigen 1, Antigen 2, and Antigen 3 were respectively subjected to enzyme-linked immunoassay (EIA) reactivity evaluation with rabbit polyclonal antibody purchased from Fitzgerald (catalog number: 70R-17581, the recognition fragment was 1-613aa of GPR37, briefly referred to as Fit). When the rabbit polyclonal antibody was diluted to 1ug/mL, the reactivity of each of the three antigens was shown in Table 1. The results showed that all three antigens had a certain activity and could be used for subsequent work.

**Table 1. Reactivity between recombinantly expressed antigens and purchased antibody**

| Antigen | Fit 1ug/mL | Blank |
|---|---|---|
| Antigen 1 | 0.7140 | 0.0100 |
| Antigen 2 | 1.4010 | 0.0160 |
| Antigen 3 | 1.2970 | 0.0110 |

### Example 2. Preparation of anti-GPR37 protein mouse monoclonal antibodies

1. Preparation of immunogen: Immunogen antigen 1, Antigen 1 prepared in Example 1 was diluted to 0.4 mg/mL with 10mmol/L PBS, and the corresponding antigen was taken and mixed with Freund's adjuvant in equal volumes according to the final concentration of 200ug/mL, thereby forming a water-in-oil emulsion. Freund's complete adjuvant was used for the initial immunization, and Freund's incomplete adjuvant was used for the booster immunization.
2. Basic immunization: 6-8 week-old BALB/c female mice was selected for subcutaneous multi-point immunization. The immunogen injection dose was 500 µL/mouse/time. The immunization interval was 2 weeks. The complete immunization program comprised 4 injections. Two weeks after the 2^{nd} and 4^{th} shots for immunization, the sera collected and isolated by the orbital blood method were subjected to indirect ELISA determination for immune titer. After the mouse serum titers confirmed that the plateau stage was reached, spleen immunization was performed with Antigen 1 at 100ug/dose 72 hours before the cell fusion.
3. Hybridoma cell screening
3.1 Preparation of macrophages as feeder cells:
(i) BALB/c mice about 6 weeks old were sacrificed by breaking neck, soaked in 75% alcohol solution for 5 minutes; taken out and placed into a sterile dish that was placed on a clean workbench in advance. The mouse posture was adjusted using hemostatic forceps, so that the abdomen faced upwards and the whole body was more stretched. The mouse placed sideways, a hemostat was used to clamp the skin at the lower part of the mouse's abdomen, and the skin was teared open by suddenly using the force in the opposite direction to fully expose the abdomen.
(ii) The peritoneum was lifted with sterile ophthalmic curved forceps, and then a 5 mL syringe was used to inject an appropriate amount of culture medium into the abdominal cavity until the abdomen was fully bulging. The syringe was put down, the hemostats was held in both hands, and the mouse contralateral limbs were lifted and gently shaken to fully infiltrate macrophages in the peritoneal cavity, then it was extracted using the same syringe and injected into the pre-prepared 1640HT medium containing 20% fetal bovine serum for later use.

3.2 Preparation of thymocytes as feeder cells:
(i) Three-week-old BALB/c mice were sacrificed by breaking neck, soaked in 75% alcohol solution for 5 minutes; taken out and placed into a sterile dish that was placed on a clean workbench in advance. The mouse posture was adjusted using hemostatic forceps, so that the abdomen faced upwards and the whole body was more stretched. The mouse was placed sideways, a hemostat was used to clamp the skin on the lower part of the mouse's chest, and the skin was teared open by suddenly using the force in the opposite direction to fully expose the chest and abdomen.
(ii) A sterile curved forceps was used with left hand to clamp the tip of sternum across the skin of the chest and abdomen, cutting was performed along the outer edge of the sternum to fully expose the thoracic diaphragm, and the clamping of the tip of the sternum was always kept to expose the chest cavity as much as possible.
(iii) A clean ophthalmic scissors was held in right hand to cut open the thoracic diaphragm to fully expose the thoracic cavity. A curved forceps was used to probe into the thoracic cavity, the root of thymus was clamped, the thymus was completely taken out and placed into a 200-mesh mesh that had been placed in a flat dish and soaked with culture medium in advance, and ground to obtain a thymus feeder cell fluid, and it was totally transferred into the above-mentioned 1640HT medium containing 20% fetal calf serum for later use.

3.3 Preparation of mouse myeloma cells: Mouse myeloma cells were resuscitated 5 days before fusion. Each fusion required approximately 6 bottles of 35 cm² mouse myeloma cells with a density of 90% to 100%.
3.4 Preparation of spleen cells:
(i) BALB/C mice to be fused were taken, blood was collected from orbit. After the blood flow stopped, the mice were sacrificed by breaking neck, soaked in 75% alcohol solution for 5 minutes, and then placed on a sterile plate in a clean bench, in right side decubitus position.
(ii) The abdominal cavity was exposed by the method described in 3.1, the abdominal cavity was opened with aseptic surgery, the spleen was taken out, placed in a 200-mesh mesh that had been placed on a dish and soaked with culture medium in advance, and ground to prepare a spleen cell suspension, and it was transferred into a 50 mL sterile centrifuge tube.
(iii) An appropriate amount, i.e., 30 to 35 mL, of RPMI-1640 culture medium was supplemented, a curved tube was used to remove obvious fat agglomerates and other impurities, centrifugation was carried out at 1500 rpm/min, 5 min/time to collect spleen cells, then the supernatant was removed by centrifugation, and the above process was repeated twice by supplementing with 30 to 35 mL of new culture medium to wash the spleen cells.
(iv) The cells were resuspended in RPMI-1640 culture medium and counted.

3.5 Cell fusion:
(i) Before fusion, 1 mL of PEG-1450, 35 mL of RPMI-1640 serum-free medium and 200 mL of pre-prepared HAT complete medium containing 20% fetal calf serum were pre-warmed to 37°C.
(ii) The prepared myeloma cells and splenocytes at a ratio of 1×10⁸ spleen cells + 1×10⁷ myeloma cells, i.e., at a ratio of about 10:1, were mixed in a 50 mL centrifuge tube, and centrifuged at 1500 rpm for 5 minutes. After centrifugation, the supernatant was discarded as much as possible by pouring, the bottom of the tube was gently tapped to loosen the cells into a paste.
(iii) 1 mL of PEG was pipetted with a 1 mL pipette and added to the centrifuge tube, gently pipetted with an elbow pipette during the addition for well mixing. The time was controlled to about 60 s, then 35 mL of pre-warmed RPMI-1640 complete culture medium was added to terminate the fusion reaction.
(iv) After standing for 1 to 5 minutes, centrifugation was carried out at 1000 rpm for 5 minutes, and the supernatant was carefully discarded. After gently tapping with finger to loose the cells, the above complete culture medium was added to fully resuspend the cells, then they were all transferred into a complete culture medium that had been added with the feeder cells in advance, mixed well, plated at 200 ul/well into a 96-well cell culture plate, and placed in a CO₂ incubator for culture.
(v) After 7 days, 15% HT complete culture medium was used to replace 100% of the cell supernatant in the well; after 7 days, the supernatant was pipetted for detection.

3.6 Screening of hybridomas: Antigen 1 was used for primary screening, and two antigens that were also fused to pET-32a were used as accessory means for differential screening, in which both antigens were from Inbermax (Catalog No.: E11024, Catalog No.: E1212), and the self-made recombinant Antigen 2 and Antigen 3 were used at the same for identification. The screening method was an indirect ELISA, and its specific steps comprised: the plate was coated with the antigen at 200 ng/mL, 100 ul per well, added with 50 uL of cell supernatant, incubated at 37°C for 30 minutes, washed 5 times with a plate washer, added with 100 ul of GAM-HRP prepared at 1:5K, incubated at 37°C for 30 minutes, washed 5 times with a plate washer, added with a chromogenic solution at 100 ul/well, incubated at 37°C for 15 minutes, added with a stop solution to terminated the reaction, and placed in a microplate reader to read signal values using dual wavelengths. Based on the experimental results, the cells in the corresponding positive wells were selected for the next step work.
3.7 Cloning of hybridoma cells: Using the limiting dilution method, the cells were first diluted in gradient at certain concentrations, and then inoculated into each well of a 96-well cell culture plate, so that only one cell grew in the well as much as possible. The hybridoma monoclonal positive cell line was repeatedly cloned at least 3 times, and were confirmed as a stable clone after the final round was 100% positive. The positive standards were as described in 3.6. The test results of the stable clone obtained by the final screening were shown in Table 2. From these results, it could be seen that antibodies 5A12 and 6D3 only recognized Antigen 1, but did not recognize Antigen 2 and extracellular segment 1-265aa antigen, so that it was initially believed that they were not extracellular segment antibodies. The remaining 10 antibodies all recognized Antigen 3, which was an extracellular segment antigen, that was, the antibodies all recognized 1-265aa, and this part of the antibody was defined as an extracellular segment antibody.

**Table 2. Cell supernatant antigen reactivity**

| No. | Name | Antigen 1 | Antigen 2 | Antigen 3 | E11024 | E1212 |
|---|---|---|---|---|---|---|
| 1 | 4A1 | 2.7630 | 1.3960 | 2.9880 | 0.0070 | 0.0140 |
| 2 | 4C10 | 3.0880 | 2.6060 | 3.1590 | 0.0220 | 0.0070 |
| 3 | 4H10-2 | 2.3510 | 3.2030 | 2.9280 | 0.0060 | 0.0130 |
| 4 | 5A2 | 2.8280 | 6.2380 | 3.3670 | 0.0080 | 0.0110 |
| 5 | 5A3 | 3.0420 | 2.5330 | 2.3940 | 0.0110 | 0.0050 |
| 6 | 5A7 | 1.7210 | 3.6170 | 3.5870 | 0.0180 | 0.0290 |
| 7 | 5A12 | 1.0920 | 0.0080 | 0.0150 | 0.0080 | 0.0070 |
| 8 | 5B11 | 3.5210 | 2.6700 | 3.2370 | 0.0090 | 0.0060 |
| 9 | 5H11 | 3.3050 | 3.0240 | 2.8060 | 0.0080 | 0.0110 |
| 10 | 6D1 | 3.5230 | 3.2960 | 3.4530 | 0.0140 | 0.0130 |
| 11 | 6D2 | 3.3650 | 2.8140 | 3.5390 | 0.0090 | 0.0100 |
| 12 | 6D3 | 1.5090 | 0.0180 | 0.0190 | 0.0180 | 0.0090 |

3.8 Antibody typing: 50 ul/well of stable cell line supernatant was added in parallel to the plate coated with Antigen 1, the plate was placed in a 37°C incubator for 30 minutes, washed 5 times, dried by beating and then added with IgG1, IgG2a, IgG2b, IgG3, and IgM typing enzymes at 50 ul/well, respectively. After reacting for 30 minutes, the plate was washed and added with a chromogenic solution at 100 ul/well. After 15 minutes, a stop solution was used for termination, and the plate was read. The typing results were shown in Table 3.

**Table 3. Cell supernatant typing results**

| No. | Name | IgG1 | IgG2a | IgG2b | IgG3 | IgM |
|---|---|---|---|---|---|---|
| 1 | 4A1 | 0.0090 | 0.0160 | 3.4700 | 0.0120 | 0.0180 |
| 2 | 4C10 | 0.0110 | 0.0110 | 3.4970 | 0.0100 | 0.0090 |
| 3 | 4H10-2 | 0.0140 | 0.0050 | 3.4650 | 0.0080 | -0.0040 |
| 4 | 5A2 | 0.0070 | 3.3690 | 0.0240 | 0.0010 | 0.0040 |
| 5 | 5A3 | 0.0200 | 2.5890 | 0.0120 | 0.0110 | 0.0040 |
| 6 | 5A7 | 0.0120 | 3.1670 | 0.0570 | 0.0170 | 0.0170 |
| 7 | 5A12 | 0.0050 | 3.3610 | 0.0280 | 0.0060 | 0.0060 |
| 8 | 5B11 | 0.0070 | 3.1080 | 0.0060 | 0.0010 | -0.0020 |
| 9 | 5H11 | 0.0130 | 0.0090 | 3.5220 | 0.0120 | 0.0050 |
| 10 | 6D1 | 0.0120 | 0.0130 | 3.4970 | 0.0030 | -0.0010 |
| 11 | 6D2 | 0.1050 | 2.9160 | 0.0200 | 0.0140 | 0.0090 |
| 12 | 6D3 | 0.0100 | 2.9360 | 0.0810 | 0.0140 | 0.0200 |

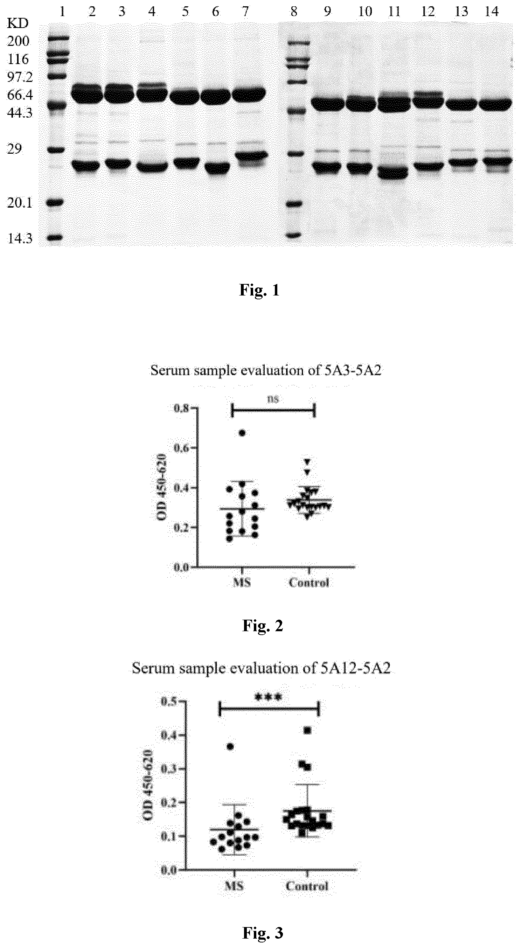

4. Preparation of monoclonal antibodies
4.1 Production of monoclonal antibody ascites
   After 1 week of sensitization of BALB/c mice, the stable hybridoma cells in the logarithmic growth phase were resuspended, and centrifuged at 1500 rpm for 5 minutes to collect the cells. The precipitated cells were suspended in serum-free culture medium, and the cell number was adjusted to (1-2)×10⁶/mL, each mouse was injected intraperitoneally with 0.5 mL for ascites induction. After 7 to 10 days, when the abdomen of the mice was obviously enlarged, the ascites was collected. The collected ascitic fluid was centrifuged in a centrifuge tube at 12,000 rpm for 10 minutes. After centrifugation, the supernatant was collected for later use.
4.2 Preparation of monoclonal antibodies
   The collected ascites was purified by ammonium sulfate precipitation and Protein A affinity chromatography (purchased from GE, USA). The purified monoclonal antibodies were identified by 12% SDS-PAGE gel running. The results were shown in Fig. 1 (where, Lane 1: Marker, Lane 2: 4A1, Lane 3: 4C10, Lane 4: 4H10-2, Lane 5: 5A2, Lane 6: 5A3, Lane 7: 5A7, Lane 8: Marker, Lane 9: 5A12, Lane 10: 5B11, Lane 11: 5H11, Lane 12:6D1, Lane 13: 6D2, Lane 14: 6D3), and they all hade a purity of 90% or more, proving that the prepared monoclonal antibodies had good purity. After sequencing, the sequence information of the obtained antibodies was shown in Table 1.

### Example 3. Pair screening of anti-GPR37 protein mouse monoclonal antibodies by enzyme-linked immunoassay (EIA)

Using 20 mmol/L PB7.4 as the coating buffer solution, the first antibody was coated on a irradiation plate at 5 ug/mL, 100 ul/well, 37°C for 2 hours; using horseradish peroxidase HRP, the second antibody was labeled at a ratio of 1:1, the final concentration of labeling was 1 mg/mL, and the labeling concentration used was 1:500. The 12 antibodies obtained in Example 2 were paired sequentially to perform a 12*12 antibody orthogonal pairing evaluation. The evaluation samples were 100 ng/mL Antigen 1, 100 ng/mL Antigen 3, 10-fold diluted normal human serum, and diluent 10 mmol/L PBS. The evaluation results were shown in Table 4. There were three pair screening criteria, and only pair that met all three criteria could be further evaluated. The specific criteria were as follows:
1. At least one of Antigen 1 or Antigen 3 must be detected. In the cases of extracellular segment antibody pairs, the detection results of Antigen 1 and Antigen 3 must be OD>1. In the cases that an extracellular segment paired with a non-extracellular segment antibody, or a non-extracellular segment paired with a non-extracellular segment antibody, it was considered to be detected as long as the antigen detection was significantly different from that of the background, and the ratio of Antigen 1/PBS was higher than 2; (about the extracellular segment and non-extracellular segment antibodies, see Section 3.7 for details)
2. Normal human serum samples must have a test result of OD>1;
   (Explanation of normal human serum sample testing standards: The purpose of establishing prototype reagents was to facilitate the screening of diseases through blood sample screening. Therefore, for blood samples, positive detection was a necessary condition. At the same time, the 10-fold dilution standard was set. On the one hand, when the level of a target contained in normal humans was uncertain, it could be possible that the level of the target was higher in normal people but lower in patients, then higher requirements were put forward for the detection sensitivity of kits. Thus, if it could still be detected in a 10-fold diluted normal person sample, the corresponding signal values could be surely detected in patient samples. On the other hand, 10-fold dilution could further reduce the amount of samples. The type of disease explored in this patent was a rare disease, and the samples were scarce, so that each case was important for research. We hoped to further save the use of samples by improving the sensitivity of reagents to conduct more researches. The sample dilution factor could also be 5 times, or even 100 times, 1000 times. We had comprehensively considered the convenience to perform experiments, reliability of results, and whether it covered possible situations, to choose 10 times as the standard);
3. The PBS detection result must be <0.1. If an extracellular segment paired with a non-extracellular segment antibody, or a non-extracellular segment paired with a non-extracellular segment antibody, when the ratio of Antigen 1/PBS signal was higher than 2, the detection standard could be appropriately broadened to OD<0.2 or less; (about extracellular and non-extracellular antibodies, see 3.7 for details)

By screening based on the above standards, in the 12*12 orthogonal pairing, 4 antibody pairs 5A3-5A2, 5A12-5A2, 6D2-5A2, 6D3-5A2 met the requirements, and these 4 antibody pairs all contained antibody 5A2, suggesting that this antibody had great application potential in the manufacture of a reagent or a combination of reagents for detecting GPR37.

The pairs 5A3-5A2 and 6D2-5A2 could simultaneously detect Antigen 1 and Antigen 3, suggesting that the smallest target for pair detection was the antigen extracellular segment, that was 1-265aa, while 5A12-5A2 and 6D3-5A2 could detect only Antigen 1, suggesting that the target for pair detection was the full-length protein fragment. It was initially believed that these four pairs could detect antigens and samples at the same time, had higher detection sensitivity in comparison, and could be further evaluated for the significance of clinical sample detection.

**Table 4. Results of antibody orthogonal pairing**

| No. | Coating | Label | Antigen 1 | Antigen 3 | Normal human serum | PBS |
|---|---|---|---|---|---|---|
| 1 | 4A1 | 4A1 | 0.531 | 0.456 | 0.08 | 0.027 |
| 2 | 4C10 | 4A1 | 0.437 | 0.237 | 0.066 | 0.026 |
| 3 | 4H10-2 | 4A1 | 0.545 | 0.453 | 0.04 | 0.026 |
| 4 | 5A2 | 4A1 | 0.094 | 0.089 | 1.165 | 0.026 |
| 5 | 5A3 | 4A1 | 0.476 | 0.406 | 2.767 | 0.033 |
| 6 | 5A7 | 4A1 | 0.697 | 0.614 | 3.072 | 0.106 |
| 7 | 5A12 | 4A1 | 0.184 | 0.037 | 3.06 | 0.057 |
| 8 | 5B11 | 4A1 | 0.143 | 0.143 | 0.9 | 0.021 |
| 9 | 5H11 | 4A1 | 0.784 | 0.626 | 0.053 | 0.04 |
| 10 | 6D1 | 4A1 | 0.397 | 0.429 | 0.074 | 0.025 |
| 11 | 6D2 | 4A1 | 0.689 | 0.525 | 3.012 | 0.029 |
| 12 | 6D3 | 4A1 | 0.118 | 0.027 | 2.992 | 0.02 |
| 13 | 4A1 | 4C10 | 0.146 | 0.1 | 0.04 | 0.019 |
| 14 | 4C10 | 4C10 | 0.14 | 0.107 | 0.04 | 0.022 |
| 15 | 4H10-2 | 4C10 | 0.132 | 0.077 | 0.04 | 0.02 |
| 16 | 5A2 | 4C10 | 0.035 | 0.024 | 0.239 | 0.018 |
| 17 | 5A3 | 4C10 | 0.089 | 0.107 | 0.417 | 0.02 |
| 18 | 5A7 | 4C10 | 0.191 | 0.146 | 1.085 | 0.058 |
| 19 | 5A12 | 4C10 | 0.099 | 0.037 | 1.842 | 0.043 |
| 20 | 5B11 | 4C10 | 0.048 | 0.026 | 0.155 | 0.022 |
| 21 | 5H11 | 4C10 | 0.249 | 0.18 | 0.041 | 0.023 |
| 22 | 6D1 | 4C10 | 0.175 | 0.112 | 0.053 | 0.021 |
| 23 | 6D2 | 4C10 | 0.273 | 0.166 | 1.446 | 0.03 |
| 24 | 6D3 | 4C10 | 0.066 | 0.026 | 1.738 | 0.027 |
| 25 | 4A1 | 4H10-2 | 0.185 | 0.125 | 0.087 | 0.039 |
| 26 | 4C10 | 4H10-2 | 0.186 | 0.089 | 0.056 | 0.027 |
| 27 | 4H10-2 | 4H10-2 | 0.108 | 0.075 | 0.048 | 0.032 |
| 28 | 5A2 | 4H10-2 | 0.051 | 0.031 | 0.131 | 0.035 |
| 29 | 5A3 | 4H10-2 | 0.098 | 0.078 | 0.356 | 0.031 |
| 30 | 5A7 | 4H10-2 | 0.233 | 0.141 | 0.761 | 0.056 |
| 31 | 5A12 | 4H10-2 | 0.077 | 0.037 | 0.852 | 0.036 |
| 32 | 5B11 | 4H10-2 | 0.067 | 0.036 | 0.189 | 0.025 |
| 33 | 5H11 | 4H10-2 | 0.196 | 0.139 | 0.046 | 0.031 |
| 34 | 6D1 | 4H10-2 | 0.193 | 0.122 | 0.096 | 0.038 |
| 35 | 6D2 | 4H10-2 | 0.304 | 0.183 | 0.893 | 0.036 |
| 36 | 6D3 | 4H10-2 | 0.065 | 0.031 | 0.871 | 0.032 |
| 37 | 4A1 | 5A2 | 2.513 | 1.251 | 0.144 | 0.216 |
| 38 | 4C10 | 5A2 | 2.626 | 1.135 | 0.126 | 0.144 |
| 39 | 4H10-2 | 5A2 | 2.472 | 1.223 | 0.182 | 0.271 |
| 40 | 5A2 | 5A2 | 1.928 | 1.675 | 3.235 | 1.998 |
| 41 | 5A3 | 5A2 | 2.515 | 1.159 | 3.247 | 0.095 |
| 42 | 5A7 | 5A2 | 2.74 | 1.793 | 3.171 | 0.465 |
| 43 | 5A12 | 5A2 | 0.896 | 0.158 | 3.093 | 0.159 |
| 44 | 5B11 | 5A2 | 1.49 | 1.242 | 3.137 | 1.155 |
| 45 | 5H11 | 5A2 | 2.801 | 2.139 | 0.17 | 0.178 |
| 46 | 6D1 | 5A2 | 2.38 | 1.99 | 0.456 | 1.117 |
| 47 | 6D2 | 5A2 | 2.731 | 2.117 | 3.049 | 0.113 |
| 48 | 6D3 | 5A2 | 0.439 | 0.042 | 3.087 | 0.063 |
| 49 | 4A1 | 5A3 | 0.471 | 0.341 | 0.172 | 0.033 |
| 50 | 4C10 | 5A3 | 0.58 | 0.328 | 0.274 | 0.052 |
| 51 | 4H10-2 | 5A3 | 0.29 | 0.237 | 0.115 | 0.034 |
| 52 | 5A2 | 5A3 | 0.274 | 0.226 | 3.386 | 0.129 |
| 53 | 5A3 | 5A3 | 0.241 | 0.207 | 3.4 | 0.1 |
| 54 | 5A7 | 5A3 | 0.533 | 0.347 | 3.269 | 0.3 |
| 55 | 5A12 | 5A3 | 0.13 | 0.087 | 3.258 | 0.078 |
| 56 | 5B11 | 5A3 | 0.204 | 0.153 | 3.156 | 0.099 |
| 57 | 5H11 | 5A3 | 0.22 | 0.158 | 0.092 | 0.055 |
| 58 | 6D1 | 5A3 | 0.242 | 0.184 | 0.157 | 0.052 |
| 59 | 6D2 | 5A3 | 0.438 | 0.268 | 3.241 | 0.024 |
| 60 | 6D3 | 5A3 | 0.057 | 0.016 | 3.3 | 0.017 |
| 61 | 4A1 | 5A7 | 0.199 | 0.257 | 0.036 | 0.011 |
| 62 | 4C10 | 5A7 | 0.202 | 0.187 | 0.036 | 0.008 |
| 63 | 4H10-2 | 5A7 | 0.187 | 0.201 | 0.019 | 0.009 |
| 64 | 5A2 | 5A7 | 0.054 | 0.041 | 0.231 | 0.009 |
| 65 | 5A3 | 5A7 | 0.151 | 0.243 | 0.384 | 0.01 |
| 66 | 5A7 | 5A7 | 0.121 | 0.165 | 0.595 | 0.019 |
| 67 | 5A12 | 5A7 | 0.055 | 0.016 | 0.579 | 0.013 |
| 68 | 5B11 | 5A7 | 0.074 | 0.053 | 0.16 | 0.009 |
| 69 | 5H11 | 5A7 | 0.247 | 0.192 | 0.013 | 0.013 |
| 70 | 6D1 | 5A7 | 0.13 | 0.171 | 0.031 | 0.011 |
| 71 | 6D2 | 5A7 | 0.181 | 0.178 | 0.921 | 0.014 |
| 72 | 6D3 | 5A7 | 0.05 | 0.015 | 0.89 | 0.014 |
| 73 | 4A1 | 5A12 | 0.136 | 0.01 | 0.051 | 0.012 |
| 74 | 4C10 | 5A12 | 0.193 | 0.038 | 0.138 | 0.034 |
| 75 | 4H10-2 | 5A12 | 0.077 | 0.013 | 0.031 | 0.009 |
| 76 | 5A2 | 5A12 | 0.142 | 0.016 | 3.182 | 0.018 |
| 77 | 5A3 | 5A12 | 0.103 | 0.013 | 3.266 | 0.014 |
| 78 | 5A7 | 5A12 | 0.206 | 0.077 | 3.406 | 0.065 |
| 79 | 5A12 | 5A12 | 0.024 | 0.033 | 3.264 | 0.027 |
| 80 | 5B11 | 5A12 | 0.122 | 0.011 | 3.073 | 0.011 |
| 81 | 5H11 | 5A12 | 0.192 | 0.014 | 0.039 | 0.011 |
| 82 | 6D1 | 5A12 | 0.161 | 0.013 | 0.054 | 0.014 |
| 83 | 6D2 | 5A12 | 0.156 | 0.02 | 3.263 | 0.012 |
| 84 | 6D3 | 5A12 | 0.021 | 0.021 | 3.335 | 0.015 |
| 85 | 4A1 | 5B11 | 0.37 | 0.461 | 0.039 | 0.012 |
| 86 | 4C10 | 5B11 | 0.358 | 0.41 | 0.028 | 0.011 |
| 87 | 4H10-2 | 5B11 | 0.318 | 0.372 | 0.146 | 0.006 |
| 88 | 5A2 | 5B11 | 0.022 | 0.017 | 0.423 | 0.011 |
| 89 | 5A3 | 5B11 | 0.223 | 0.341 | 0.736 | 0.022 |
| 90 | 5A7 | 5B11 | 0.194 | 0.276 | 0.433 | 0.014 |
| 91 | 5A12 | 5B11 | 0.075 | 0.013 | 0.714 | 0.009 |
| 92 | 5B11 | 5B11 | 0.049 | 0.051 | 0.284 | 0.017 |
| 93 | 5H11 | 5B11 | 0.4 | 0.394 | 0.023 | 0.021 |
| 94 | 6D1 | 5B11 | 0.127 | 0.235 | 0.032 | 0.005 |
| 95 | 6D2 | 5B11 | 0.288 | 0.432 | 1.012 | 0.011 |
| 96 | 6D3 | 5B11 | 0.068 | 0.009 | 1.357 | 0.008 |
| 97 | 4A1 | 5H11 | 1.692 | 0.025 | 0.086 | 0.02 |
| 98 | 4C10 | 5H11 | 1.581 | 0.028 | 0.056 | 0.017 |
| 99 | 4H10-2 | 5H11 | 1.019 | 0.014 | 0.044 | 0.015 |
| 100 | 5A2 | 5H11 | 1.486 | 0.015 | 0.071 | 0.019 |
| 101 | 5A3 | 5H11 | 1.34 | 0.021 | 0.089 | 0.02 |
| 102 | 5A7 | 5H11 | 1.451 | 0.027 | 0.08 | 0.024 |
| 103 | 5A12 | 5H11 | 0.158 | 0.009 | 0.078 | 0.009 |
| 104 | 5B11 | 5H11 | 1.277 | 0.014 | 0.074 | 0.028 |
| 105 | 5H11 | 5H11 | 0.044 | 0.023 | 0.021 | 0.014 |
| 106 | 6D1 | 5H11 | 1.456 | 0.013 | 0.071 | 0.008 |
| 107 | 6D2 | 5H11 | 1.371 | 0.022 | 0.091 | 0.015 |
| 108 | 6D3 | 5H11 | 0.101 | 0.008 | 0.089 | 0.012 |
| 109 | 4A1 | 6D1 | 0.665 | 0.525 | 0.036 | 0.013 |
| 110 | 4C10 | 6D1 | 0.595 | 0.434 | 0.038 | 0.011 |
| 111 | 4H10-2 | 6D1 | 0.534 | 0.377 | 0.021 | 0.012 |
| 112 | 5A2 | 6D1 | 0.108 | 0.047 | 0.459 | 0.02 |
| 113 | 5A3 | 6D1 | 0.522 | 0.353 | 0.695 | 0.024 |
| 114 | 5A7 | 6D1 | 0.424 | 0.23 | 0.635 | 0.045 |
| 115 | 5A12 | 6D1 | 0.164 | 0.021 | 0.79 | 0.026 |
| 116 | 5B11 | 6D1 | 0.124 | 0.067 | 0.325 | 0.012 |
| 117 | 5H11 | 6D1 | 0.679 | 0.32 | 0.025 | 0.014 |
| 118 | 6D1 | 6D1 | 0.494 | 0.412 | 0.055 | 0.015 |
| 119 | 6D2 | 6D1 | 0.41 | 0.347 | 0.77 | 0.015 |
| 120 | 6D3 | 6D1 | 0.107 | 0.018 | 0.68 | 0.013 |
| 121 | 4A1 | 6D2 | 0.187 | 0.265 | 0.054 | 0.01 |
| 122 | 4C10 | 6D2 | 0.198 | 0.178 | 0.047 | 0.012 |
| 123 | 4H10-2 | 6D2 | 0.175 | 0.216 | 0.024 | 0.008 |
| 124 | 5A2 | 6D2 | 0.042 | 0.03 | 2.956 | 0.014 |
| 125 | 5A3 | 6D2 | 0.169 | 0.207 | 3.116 | 0.013 |
| 126 | 5A7 | 6D2 | 0.157 | 0.185 | 3.237 | 0.068 |
| 127 | 5A12 | 6D2 | 0.075 | 0.029 | 3.232 | 0.033 |
| 128 | 5B11 | 6D2 | 0.048 | 0.032 | 2.88 | 0.01 |
| 129 | 5H11 | 6D2 | 0.249 | 0.182 | 0.023 | 0.009 |
| 130 | 6D1 | 6D2 | 0.122 | 0.148 | 0.053 | 0.007 |
| 131 | 6D2 | 6D2 | 0.145 | 0.162 | 3.019 | 0.011 |
| 132 | 6D3 | 6D2 | 0.048 | 0.014 | 3.143 | 0.013 |
| 133 | 4A1 | 6D3 | 0.057 | 0.011 | 0.022 | 0.009 |
| 134 | 4C10 | 6D3 | 0.04 | 0.007 | 0.023 | 0.005 |
| 135 | 4H10-2 | 6D3 | 0.023 | 0.018 | 0.01 | 0.006 |
| 136 | 5A2 | 6D3 | 0.041 | 0.006 | 1.293 | 0.007 |
| 137 | 5A3 | 6D3 | 0.038 | 0.01 | 2.089 | 0.008 |
| 138 | 5A7 | 6D3 | 0.048 | 0.018 | 2.06 | 0.028 |
| 139 | 5A12 | 6D3 | 0.013 | 0.013 | 2.594 | 0.01 |
| 140 | 5B11 | 6D3 | 0.035 | 0.008 | 0.791 | 0.006 |
| 141 | 5H11 | 6D3 | 0.053 | 0.004 | -0.001 | 0.004 |
| 142 | 6D1 | 6D3 | 0.035 | 0.001 | 0.017 | 0.004 |
| 143 | 6D2 | 6D3 | 0.035 | 0.005 | 1.998 | 0.006 |
| 144 | 6D3 | 6D3 | 0.006 | 0.006 | 2.926 | 0.007 |

### Example 4. Evaluation of performance of GPR37 antibody pair in clinical sample analysis

### 1. Serum sample evaluation

Sera were collected from 15 cases of MS and 20 cases of normal humans, and the above 4 pairs 5A3-5A2, 5A12-5A2, 6D2-5A2 and 6D3-5A2 were used to evaluate the detection effect on these clinical samples. The specific method was as follows: all samples were diluted 20 times with 10 mmol/L PBS, added at 100 ul/well to the respectively antibody-coated enzyme plates, incubated at 37°C for 30 minutes, then the plates were washed 5 times, added with 1:500 diluted 5A2-HRP at 100 ul/well, the reaction was continued at 37°C for 30 min, the plate was washed 5 times, added with a chromogenic solution at 100 ul/well, the color development reaction was carried out at 37°C for 15 min and then terminated, the enzyme plates were read, and the detection results were subjected to statistical analysis of data using Graphpad Prism 8.0.

The detection effects of the 4 pairs for samples were shown in Fig.s 2, 3, 4 and 5 respectively. All four pairs have detection signals for all samples. Among them, the pairs 5A3-5A2 and 6D3-5A2 could not effectively discriminate MS patients from normal people. Both of pairs 5A12-5A2 and 6D2-5A2 were better when detecting patient samples, and showed a more consistent trend, that was, the detection results of MS patients were significantly lower than the detection results of normal people, suggesting that detecting GPR37 levels in serum samples could effectively discriminate between MS patients and normal people.

### 2. Cerebrospinal fluid sample evaluation

Cerebrospinal fluid samples were collected from 13 cases of NMOSD, 15 cases of MS, and 21 cases of GBS, respectively, and the above 4 pairs 5A3-5A2, 5A12-5A2, 6D2-5A2 and 6D3-5A2 were used to evaluate the detection effect on these clinical samples. The specific method was as follows: all samples were diluted 10 times with 10 mmol/L PBS, added at 100 ul/well to the respectively antibody-coated enzyme plates, and incubated at 37°C for 30 minutes, the plates were washed 5 times, added with 1: 500 diluted 5A2-HRP at 100 ul/well, the reaction was continued at 37°C for 30 minutes, the plate was washed 5 times, added with a chromogenic solution at 100 ul/well, the color development reaction was carried out at 37°C for 15 minutes and then terminated, the enzyme plates were read, and the detection results were subjected to statistical analysis of data using Graphpad Prism 8.0.

The detection results of the 4 pairs on the cerebrospinal fluid samples were shown in Fig.s 6, 7, 8 and 9. The results showed that the OD values in the detection of the pair 5A3-5A2 were lower, indicating that the detection sensitivity of the pair was lower. The 3 pairs 5A12-5A2, 6D2-5A2 and 6D3-5A2 all had good detection results on cerebrospinal fluid samples, and the statistical analysis results showed that neither pair 5A12-5A2 nor pair 6D3-5A2 could effectively discriminate between the three demyelinating diseases of NMOSD, MS and GBS, while the pairs 5A3-5A2 and 6D2-5A2 could significantly discriminate between NMOSD and MS. Especially for the pair 6D2-5A2, the further analysis showed that the pairs 5A12-5A2 and 6D3-5A2 detected the full-length form of GPR37 antigen, while the pairs 5A3-5A2 and 6D2-5A2 could detect the extracellular segment of GPR37 antigen; by combining the detection results of the two pairs on the samples, it was further speculated that the detection of GPR37 extracellular segment, especially the detection of GPR37 extracellular segment protein levels in cerebrospinal fluid samples, could be used to discriminate between NMOSD and MS diseases.

### 3. Analysis of pair performance

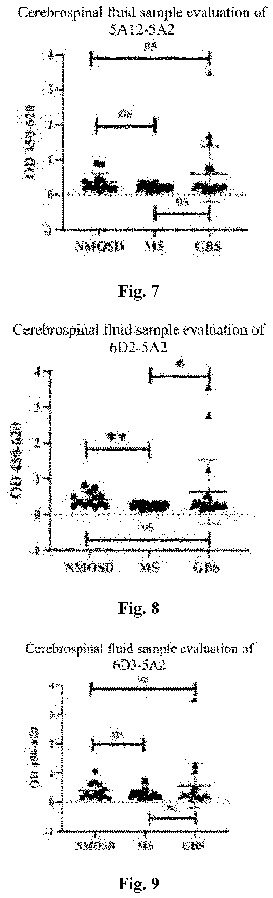

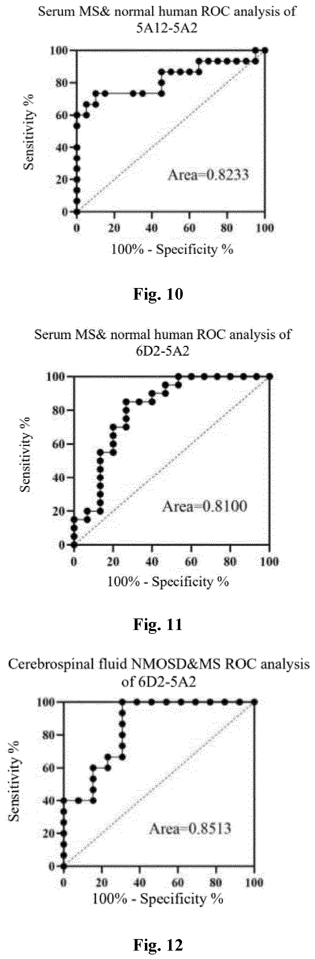

Graphpad Prism 8.0 was used to conduct ROC working curve analysis on the detection results of the pair 5A12-5A2 for MS and normal human serum samples. The results were shown in Fig. 10. The detection results of 6D2-5A2 detection for MS and normal human serum samples as well as NMOSD and MS cerebrospinal fluid samples were subjected to ROC working curve analysis, and the results were shown in Fig.s 11 and 12. The results in Fig. 10 showed that the pair 5A12-5A2 only had a good discrimination degree in detecting normal and MS sera (AUC=0.8233). The detection results of the pair 5A12-5A2 on cerebrospinal fluid samples showed that there was no significant difference in the levels of GPR37 between NMOSD and MS (as shown in the Fig. 7). Under the same circumstances, the ROC working curve of 6D2-5A2 for detecting normal people and MS sera had AUC=0.8100, showing that it could better discriminate between normal people and MS patients. Furthermore, the ROC working curve of the pair 6D2-5A2 for detecting NMOSD and MS cerebrospinal fluid samples had AUC=0.8513, indicating that this pair could well discriminate NMOSD and MS patients.

In summary, the pair of two antibodies 6D2 and 5A2, which had good binding reactivity with the extracellular segment (1-265aa) of GPR37, had outstanding detection effects. This pair could effectively discriminate MS from normal people in the detection of blood samples. Furthermore, this pair of antibodies could effectively discriminate MS and NMOSD in the detection of cerebrospinal fluid samples.

### Example 5. Identification of anti-GPR37 antibody epitope

### 1. Preparation of 101-265aa antigen

Using the synthesized GPR37 gene as a template, the nucleotide sequence encoding amino acids at positions 101 to 265 of GPR37 (the amino acid sequence was set forth in SEQ ID NO: 21) was amplified, the PCR-amplified DNA fragments underwent 1.2% agarose gel electrophoresis, and TIANGEN gel recovery kit was used for purification and recovery. Gibson assembly solution was used to assemble the recovered and purified fragments and the pCold TF vector, in which the molar ratio of the target fragment to the vector was 2:1 to 4:1, and the system was as follows: vector fragment: 1 µL; DNA fragment of the amino acids at the positions 101 to 265 of GPR37: 4 µL; Gibson assembly solution: 5 ul, were prepared, vortexed and mixed well, and the reaction was carried out in a 50°C water bath for 40 minutes. Then the conjugated system was transformed into *E. coli* BL21.

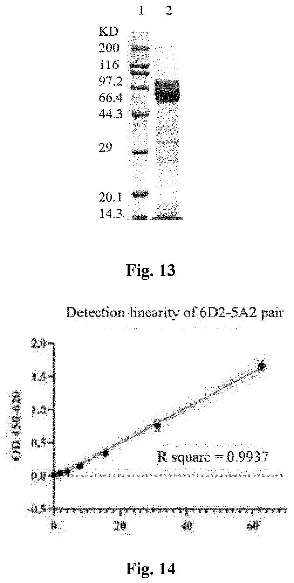

The competent BL21 was taken, placed on an ice box for 10 minutes for thawing, added with the conjugate, incubated on ice for 30 minutes, heated for shocking at 42°C for 90 seconds, placed immediately on ice for 5 minutes, then added with 900 ul of LB culture medium to the EP tube, placed in a 37°C constant-temperature shaker and cultured for 40 minutes. The cultured bacterial solution was centrifuged at 5000 rpm for 2 minutes for enrichment. The supernatant was discarded, and 200 ul was remained to resuspend the bacterial cells, which were evenly coated on a prepared ampicillin-containing (100 µL /mL) LB solid medium. After standing for 5 minutes, the bacterial solution was absorbed in the solid culture medium, which was then inverted and cultured in an incubator at 37°C for overnight. On the next day, 5 single colonies were randomly picked from the LB-containing plate, placed in 5 mL of a liquid LB medium containing ampicillin (100 µL/mL) and cultured overnight. On the next day, the cultured bacterial solution was taken as a template, and subjected to PCR amplification reaction using universal primers; when the 1.2% agarose gel electrophoresis showed positive detection result, plasmids were extracted using TIANGEN plasmid extraction kit. After the sequencing was confirmed to be correct, the induced expression work for the corresponding strains was performed, and the engineering bacteria expressing the target proteins were collected. The prepared engineering bacteria were added to a lysis buffer for fragmentation. The lysis products were purified using a medium-pressure chromatography system and nickel ion chromatography to obtain protein. The protein was named by combining the corresponding fusion vector and the range of target fragment, and abbreviated as TF 101-265. The protein was recovered and identified by gel running. The results were shown in Fig. 13 (wherein, Lane 1: Marker, Lane 2: TF-101-265).

### 2. Identification of reactivity of 5A2 antibody against different fragment antigens 1-265aa (extracellular) and 101-265aa

Using PBS as a negative control, 1-265aa (Antigen 3) and 101-265aa (TF 101-265) were diluted to 100 ug/mL, then mixed with an enzyme-labeled antibody diluted to an appropriate concentration at a volume ratio of 1:1, and incubate at 37°C for 30 minutes, then transferred at 100 ul/well to an enzyme-linked reaction plate that was coated with Antigen 1 at 20 ng/well, after being incubated at 37°C for 30 minutes, the plate was washed 5 times, added with a substrate at 100 ul/well, and reacted at 37°C for 15 minutes, the reaction was terminated with a stop solution, and the values at 450-620 dual-wavelengths were read with a microplate reader. Taking the result of the PBS reaction well as 100% binding, the reactivity between the above antigen and the antibody was evaluated by calculating the effect of the above antigen in blocking the antibody to bind Antigen 1. If and only if the blocking rate reached more than 90%, the above antigen was considered to be relatively effective in blocking the antibody to bind to Antigen 1, that was, the above antigen and antibody had good reactivity, that was, the antigen epitope segment recognized by the antibody could be determined. The calculation results were shown in Table 5.

**Table 5. Identification of GPR37 fragment recognized by 5A2 strain antibody**

| Enzyme-labeled antibody | 1-265aa | 101-265aa |
|---|---|---|
| 5A2-HRP | 99% | 98% |

The results showed that the blocking effects of the 101-265aa fragment on 5A2 and the full-length antigen were basically equivalent to that of 1-265aa, further indicating that 5A2 recognized the 101-265 segment of GPR37.

### Example 6. Construction of 6D2-5A2 prototype reagent

### 1. Prototype reagent detection linearity

Antigen 1 diluted with 10 mmol/L PBS to a total of 7 concentration points of 62.50 ng/mL, 31.25 ng/mL, 15.63 ng/mL, 7.82 ng/mL, 3.91 ng/mL, 1.95 ng/mL, PBS, and blank control were each taken and added at 50 ul/well to a 6D2-coated plate, in which detection with two wells in parallel was performed. After being incubated at 37°C for 30 minutes, the plate was washed 5 times, added at 50 ul/well with 1:400 diluted 5A2-HRP, the reaction was continued at 37°C for 30 minutes, the plate was washed 5 times, added at 100 ul/well with a chromogenic solution, the color development reaction was carried out at 37°C for 15 minutes and then terminated, the coated plate was read, and the detection results were analyzed with Graphpad Prism 8.0. The results were shown in Fig. 14, which indicated that the prototype reagent had good detection linearity.

### 2. Detection sensitivity of prototype reagents

5% bovine serum blank sample was taken and added at 50 ul/well to the 6D2-coated plate, the detection was carried out on 20 repeated wells, and the average and standard deviation of the 20 results were calculated. The average + 2 times the standard deviation was the detection lower limit of the pair 6D2*5A2, the results showed that the detection sensitivity of the pair was 0.031, and the corresponding detection concentration was calculated back to 3.2 ng/mL, as shown in Table 6.

**Table 6. Detection sensitivity of prototype reagents**

| No. | Blank sample |
|---|---|
| 1 | 0.02 |
| 2 | 0.014 |
| 3 | 0.015 |
| 4 | 0.022 |
| 5 | 0.005 |
| 6 | 0.006 |
| 7 | 0.008 |
| 8 | 0.004 |
| 9 | 0.013 |
| 10 | 0 |
| 11 | 0.001 |
| 12 | -0.01 |
| 13 | 0.031 |
| 14 | 0.025 |
| 15 | 0.019 |
| 16 | 0.021 |
| 17 | 0.007 |
| 18 | 0.011 |
| 19 | 0.012 |
| 20 | 0.013 |
| Average value | 0.012 |
| Standard deviation | 0.01 |
| Average value + 2 times standard deviation | 0.031 |

### 3. Precision of prototype reagents

Antigen 1 was used as the detection antigen, two quality control concentrations of 62.5 ng/mL and 15.625 ng/mL were selected, and the detection was repeated 10 times; the coefficient of variation (CV) of the 10 results was calculated and shown in Table 7 below, indicating that the prototype reagent had a CV controlled below 15%, and had good precision.

**Table 7. Detection precision of prototype reagents**

| Concentration | 62.5ng/mL | 15.625ng/mL |
|---|---|---|
| 1 | 1.622 | 0.357 |
| 2 | 1.524 | 0.332 |
| 3 | 1.537 | 0.321 |
| 4 | 1.374 | 0.333 |
| 5 | 1.435 | 0.334 |
| 6 | 1.34 | 0.3 |
| 7 | 1.418 | 0.301 |
| 8 | 1.404 | 0.299 |
| 9 | 1.684 | 0.332 |
| 10 | 1.716 | 0.307 |
| CV | 9% | 6% |

### 4. Stability of prototype reagents

The coating and labeling of the prototype reagent components were baked at 37°C for 3 days. The prototype reagent was stored at 4°C as a control. The signal deviations of 2 quality control points of 62.5 ng/mL and 15.625 ng/mL were detected. The results showed that the signal deviations were all within 15%, indicating that the key components of the prototype reagent had good thermal stability (the quality control product should to be freeze-stored), as shown in Table 8.

**Table 8. Thermal stability of prototype reagent coating and labeling**

| Activity deviation | 62.5 ng/mL | | 31.125 ng/mL | |
|---|---|---|---|---|
| Coating deviation | -4% | -1% | -11% | -9% |
| Labeling deviation | -1% | 4% | -1% | 0% |

Although the specific embodiments of the present invention have been described in detail, those skilled in the art will understand that various modifications and changes can be made to the details based on all teachings that have been disclosed, and these changes are within the protection scope of the present invention. The full scope of the present invention is given by the appended claims and any equivalents thereof.

## Claims

1. A kit, which is used for detecting whether a subject suffers from multiple sclerosis (MS) and/or differentiating between neuromyelitis optica spectrum disorders (NMOSD) and MS, wherein the kit comprises a reagent or a combination of reagents for determining a level of a biomarker in a biological sample, and the biomarker is GPR37 or a fragment thereof.

2. The kit according to claim 1, wherein the level of the biomarker is a protein level or mRNA level of the biomarker;
preferably, the fragment of GPR37 comprises the amino acid residues at positions 1 to 265 of the amino acid sequence of GPR37;
preferably, the fragment of GPR37 comprises the amino acid residues at positions 101 to 265 of the amino acid sequence of GPR37;
preferably, the GPR37 has the amino acid sequence as set forth in SEQ ID NO: 1;
preferably, the fragment of GPR37 has the amino acid sequence as set forth in SEQ ID NO: 3 or 21;
preferably, the biological sample is a blood sample (e.g., whole blood, plasma, or serum), an excretion (e.g., urine), a secretion (e.g., sweat, tears) or a cerebrospinal fluid obtained from a subject;
preferably, the kit is used to discriminate between subjects suffering from MS and those suffering from NMOSD.

3. The kit according to claim 1 or 2, wherein the level of the biomarker is a protein level of the biomarker;
preferably, the reagent or combination of reagents is used to determine the level of the biomarker in the biological sample by an immunological assay;
preferably, the immunological assay is selected from the group consisting of ELISA assay, Western blotting, surface plasmon resonance method, and Elispot assay;
preferably, the reagent or combination of reagents comprises: a first antibody and/or a second antibody against GPR37 or a fragment thereof.

4. The kit according to claim 1 or 2, wherein the level of the biomarker is a level of an mRNA encoding the biomarker;
preferably, the reagent or combination of reagents is used to determine the level of the biomarker in the biological sample by quantitative PCR;
preferably, the reagent or combination of reagents comprises a primer and/or probe capable of quantifying the level of the mRNA encoding GPR37 or a fragment thereof.

5. The kit according to any one of claims 1 to 4, wherein the kit comprises: (i) a first antibody or a conjugate thereof, or (ii) a second antibody or a conjugate thereof, or (iii) their combination;
wherein, the first antibody and/or the second antibody are antibodies or antigen-binding fragments that specifically bind to GPR37 or fragments thereof;
preferably, the kit is used to discriminate between healthy subjects, subjects suffering from MS and subjects suffering from NMOSD.

6. The kit according to claim 5, wherein the first antibody comprises: the following 3 heavy chain CDRs: a VH CDR1 as set forth in SEQ ID NO: 5, a VH CDR2 as set forth in SEQ ID NO: 6, a VH CDR3 as set forth in SEQ ID NO: 7; and, the following 3 light chain CDRs: a VL CDR1 as set forth in SEQ ID NO: 8, a VL CDR2 as set forth in SEQ ID NO: 9, a VL CDR3 as set forth in SEQ ID NO: 10;
preferably, the first antibody comprises: a VH as set forth in SEQ ID NO: 11; and a VL as set forth in SEQ ID NO: 12;
preferably, the conjugate of the first antibody comprises the first antibody, and a detectable label linked to the first antibody;
preferably, the detectable label is selected from the group consisting of enzyme (e.g., horseradish peroxidase, alkaline phosphatase), chemiluminescent reagent (e.g., acridinium ester, luminol and derivative thereof, ruthenium derivative), fluorescent dye (e.g., fluorescein, fluorescent protein), radionuclide or biotin;
preferably, the detectable label is horseradish peroxidase.

7. The kit according to claim 5 or 6, wherein the second antibody comprises: the following 3 heavy chain CDRs: a VH CDR1 as set forth in SEQ ID NO: 13, a VH CDR2 as set forth in SEQ ID NO: 14, a VH CDR3 as set forth in SEQ ID NO: 15; and, the following 3 light chain CDRs: a VL CDR1 as set forth in SEQ ID NO: 16, a VL CDR2 as set forth in SEQ ID NO: 17, a VL CDR3 as set forth in SEQ ID NO: 18;
preferably, the second antibody comprises: a VH as set forth in SEQ ID NO: 19; and a VL as set forth in SEQ ID NO: 20;
preferably, the conjugate of the second antibody comprises the second antibody, and a detectable label linked to the second antibody;
preferably, the detectable label is selected from the group consisting of enzyme (e.g., horseradish peroxidase, alkaline phosphatase), chemiluminescent reagent (e.g., acridinium ester, luminol and derivative thereof, ruthenium derivative), fluorescent dye (e.g., fluorescein, fluorescent protein), radionuclide or biotin;
preferably, the detectable label is horseradish peroxidase.

8. Use of a reagent for determining a level of a biomarker in a biological sample in the manufacture of a kit, in which the kit is used for detecting whether a subject suffers from MS and/or discriminating NMOSD and MS; wherein, the biomarker is GPR37 or a fragment thereof;
preferably, the level of the biomarker is a protein level or mRNA level of the biomarker;
preferably, the fragment of GPR37 comprises the amino acid residues at positions 1 to 265 of the amino acid sequence of GPR37;
preferably, the fragment of GPR37 comprises the amino acid residues at positions 101 to 265 of the amino acid sequence of GPR37;
preferably, the GPR37 has the amino acid sequence as set forth in SEQ ID NO: 1;
preferably, the fragment of GPR37 has the amino acid sequence as set forth in SEQ ID NO: 3 or 21;
preferably, the biological sample is a blood sample (e.g., whole blood, plasma or serum), an excretion (e.g., urine), a secretion (e.g., sweat, tears) or a cerebrospinal fluid obtained from the subject;
preferably, the kit is used to discriminate between subjects suffering from MS and those suffering from NMOSD.

9. The use according to claim 8, wherein the level of the biomarker is the protein level of the biomarker;
preferably, the reagent or combination of reagents is used to determine the level of the biomarker in the biological sample by an immunological assay;
preferably, the immunological assay is selected from the group consisting of ELISA assay, Western blotting, surface plasmon resonance method, and Elispot assay;
preferably, the reagent or combination of reagents comprises: a first antibody and/or a second antibody against GPR37 or a fragment thereof.

10. The use according to claim 8, wherein the level of the biomarker is the level of an mRNA encoding the biomarker;
preferably, the reagent or combination of reagents is used to determine the level of the biomarker in the biological sample by quantitative PCR;
preferably, the reagent or combination of reagents comprises a primer and/or probe capable of quantifying the level of the mRNA encoding GPR37 or a fragment thereof.

11. The use according to any one of claims 8 to 10, wherein the kit comprises: (i) a first antibody or a conjugate thereof, or (ii) a second antibody or a conjugate thereof, or (iii) their combination;
wherein, the first antibody and/or the second antibody are antibodies or antigen-binding fragments that specifically bind to G protein-coupled receptor 37 (GPR37) or fragments thereof;
preferably, the kit is used to discriminate between healthy subjects, subjects suffering from MS and subjects suffering from NMOSD.

12. The use according to claim 11, wherein the first antibody comprises: the following 3 heavy chain CDRs: a VH CDR1 as set forth in SEQ ID NO: 5, a VH CDR2 as set forth in SEQ ID NO: 6, a VH CDR3 as set forth in SEQ ID NO: 7; and, the following 3 light chain CDRs: a VL CDR1 as set forth in SEQ ID NO: 8, a VL CDR2 as set forth in SEQ ID NO: 9, a VL CDR3 as set forth in SEQ ID NO: 10;
preferably, the first antibody comprises: a VH as set forth in SEQ ID NO: 11; and a VL as set forth in SEQ ID NO: 12;
preferably, the conjugate of the first antibody comprises the first antibody, and a detectable label linked to the first antibody;
preferably, the detectable label is selected from the group consisting of enzyme (e.g., horseradish peroxidase, alkaline phosphatase), chemiluminescent reagent (e.g., acridinium ester, luminol and derivative thereof, ruthenium derivative), fluorescent dye (e.g., fluorescein, fluorescent protein), radionuclide or biotin;
preferably, the detectable label is horseradish peroxidase.

13. The use according to claim 11 or 12, wherein the second antibody comprises: the following 3 heavy chain CDRs: a VH CDR1 as set forth in SEQ ID NO: 13, a VH CDR2 as set forth in SEQ ID NO: 14, a VH CDR3 as set forth in SEQ ID NO: 15; and, the following 3 light chain CDRs: a VL CDR1 as set forth in SEQ ID NO: 16, a VL CDR2 as set forth in SEQ ID NO: 17, a VL CDR3 as set forth in SEQ ID NO: 18;
preferably, the second antibody comprises: a VH as set forth in SEQ ID NO: 19; and a VL as set forth in SEQ ID NO: 20;
preferably, the conjugate of the second antibody comprises the second antibody, and a detectable label linked to the second antibody;
preferably, the detectable label is selected from the group consisting of enzyme (e.g., horseradish peroxidase, alkaline phosphatase), chemiluminescent reagent (e.g., acridinium ester, luminol and derivative thereof, ruthenium derivative), fluorescent dye (e.g., fluorescein, fluorescent protein), radionuclide or biotin;
preferably, the detectable label is horseradish peroxidase.

14. A method for screening a reagent or a combination of agents that is capable of being used to detect whether a subject suffers from MS, and/or capable of discriminating NMOSD and MS, the method comprising:
(1) providing a biological sample containing GPR37 or a fragment thereof obtained from a subject, and contacting a reagent or combination of reagents with the biological sample;
(2) detecting whether the reagent or combination of reagents forms a complex with GPR37 or a fragment therein contained in the biological sample;
(3) determining that when the complex is formed in step (2), the reagent or combination of reagents is capable of being used to detect whether the subject suffers from MS, and/or capable of discriminating NMOSD and MS;
preferably, the fragment of GPR37 comprises the amino acid residues at positions 1 to 265 of the amino acid sequence of GPR37;
preferably, the fragment of GPR37 comprises the amino acid residues at positions 101 to 265 of the amino acid sequence of GPR37;
preferably, the GPR37 has the amino acid sequence as set forth in SEQ ID NO: 1;
preferably, the fragment of GPR37 has the amino acid sequence as set forth in SEQ ID NO: 3 or 21;
preferably, the biological sample is a blood sample (e.g., whole blood, plasma or serum), an excretion (e.g., urine), a secretion (e.g., sweat, tears) or a cerebrospinal fluid obtained from the subject;
preferably, the reagent or combination of reagents is used to discriminate subjects suffering from MS and those suffering from NMOSD;
preferably, the reagent or combination of reagents is an antibody or conjugate thereof capable of specifically binding to GPR37 or a fragment thereof;
preferably, the conjugate comprises the antibody, and a detectable label linked to the antibody;
preferably, the detectable label is selected from the group consisting of enzyme (e.g., horseradish peroxidase, alkaline phosphatase), chemiluminescent reagent (e.g., acridinium ester, luminol and derivative thereof, ruthenium derivative), fluorescent dye (e.g., fluorescein, fluorescent protein), radionuclide or biotin;
preferably, the detectable label is horseradish peroxidase.
